# EUROPEAN PATENT APPLICATION

(11) **EP 4 822 355 A2**
(43) Date of publication of application: **14.10.2026**
(21) Application number: 26188419.1
(22) Date of filing: 28.05.2019
(51) Int. Cl.: G01N 33/68

(54) **HSP70 PROTEIN LEVELS IN PBMC SAMPLES AS BIOMARKER FOR DISEASE**

(30) Priority: 28.05.2018 EP 18174576
(62) Document number(s) of the earlier application(s) in accordance with Art. 76 EPC: 19726029.2 / 3 803 406
(71) Applicant: Zevra Denmark A/S, 2000 Frederiksberg (DK)
(72) Inventor: Jensen, Thomas Kirkegaard, 2610 Rødovre (DK); Ingemann, Linda, 2870 Dyssegård (DK)
(74) Representative: Høiberg P/S

(57) **Abstract**

Disclosed herein are methods based on the identification of reduced Hsp70 levels in PBMC samples serving as a biomarker for diseases presenting with a reduced level of Hsp70, such as lysosomal storage diseases, neurodegenerative diseases and muscular diseases.

## Description

### Technical field

The present invention relates to the finding that Heat Shock Protein 70 (Hsp70) is markedly reduced in peripheral blood mononuclear cell (PBMC) patient samples of the lysosomal storage disease NPC, and hence the previously observed reduced level of Hsp70 observed in pathologically afflicted disease tissue, including brain and liver tissue, directly translate into low levels of Hsp70 protein in PBMC samples, providing a simple detection means of Hsp70 levels.

### Background

Hsp70 proteins, one of the most extensively studied families of heat shock proteins (HSPs), are synthesized in all eukaryotic cells. Hsp70 proteins have a broad spectrum of chaperone functions and provide the normal course of many intracellular processes. In addition, they are involved in the cell resistance against stress; in particular, they prevent protein aggregation and facilitate the elimination of proteins damaged under stress conditions.

Hsp70 proteins are induced by stress and heat shock, and multiple reports have documented a correlation between Hsp70 expression and disease or other conditions of stress and exercise. For instance, elevated circulating levels of Hsp70 (in plasma or serum) are identified in cardiovascular disease (heart failure after acute myocardial infarction, peripheral artery disease), cancer patients (e.g. small cell lung cancer, cholangiocarcinoma, head and neck cancer, pancreatic cancer), preeclampsia and pathological pregnancies, gestational diabetes mellitus, polycystic ovary syndrome, inflammatory conditions, asthma and frailty in elderly patients. Such elevated circulating levels Hsp70 levels are found to predict disease progression and an unfavourable clinical outcome.

Under certain pathological conditions the protein quality control machinery is not sufficient to prevent the accumulation of misfolded proteins. Hsp70 functions as a chaperone and protects neurons from protein aggregation and toxicity. A common feature among various neurodegenerative diseases, including Alzheimer disease (AD), Parkinson disease (PD), amyotrophic lateral sclerosis (ALS), and the inheritable polyglutamine (PolyQ) diseases (e.g., Huntington disease (HD); spinocerebellar ataxia (SCA) type 1, 2, 3, 6, 7, and 17; spinobulbar muscular atrophy (SBMA); dentatorubral pallidoluysian atrophy (DRPLA)) is the accumulation and deposition of misfolded proteins in the brain (inside and outside neurons) and selective neuronal loss in the central nervous system (CNS). For all of these conformational/misfolding diseases, misfolded proteins are considered a common therapeutic target, and many studies have focused on the neuroprotective role of HSPs.

The worldwide incidence of neurodegenerative diseases is high. As neurodegenerative diseases disproportionately affect older individuals, disease-related morbidity has increased along with the general increase in longevity. An understanding of the underlying mechanisms that lead to neurodegeneration is key to identifying methods of prevention and treatment. Investigators have observed protective effects of HSPs induced by preconditioning, overexpression, or drugs in a variety of models of brain disease. Experimental data suggest that manipulation of the cellular stress response, including the provision of Hsp70, may offer strategies to protect the brain during progression of neurodegenerative disease.

The lysosomal storage diseases (LSD) are a rare group of diseases, characterized by the accumulation of substances in the lysosomal compartment and resulting destabilization hereof, with a resulting devastating effect for affected individuals. Substances accumulate in the lysosomal compartment due to deficiencies in the enzymes involved in their catabolism.

The majority of LSD patients are initially screened by an enzyme assay, if available, which is the most efficient method to arrive at a definitive diagnosis. In some families where the disease-causing mutation(s) is known and in certain genetic isolates, mutation analysis may be performed. As there may be numerous different mutations, sequencing of the gene encoding the particular affected protein/enzyme is sometimes necessary to confirm the diagnosis. Prenatal diagnosis may be useful when there is a known genetic risk factor.

LSDs include Niemann Pick disease, including types A, B and C. Historically, the diagnosis of Niemann Pick disease Type C (NPC) is made histopathologically, by both esterification studies and filipin staining of cultured skin fibroblasts, with most patients receiving a combination of different tests performed prior to this reliable, but costly and difficult, definitive investigation. These tests may have included: chitotriosidase measurements, white cell enzyme studies to exclude other lysosomal storage diseases, and fluorescent and electron microscopy of both bone marrow aspirate and liver biopsy specimens. Because of the difficulties with the filipin staining test, the most widely performed and accessible definitive diagnostic test is currently the sequencing of the NPC1 and NPC2 genes. Next-generation sequencers make this far easier to perform, especially if the genes concerned are included on a multi-gene panel appropriate for patients presenting with a certain disease phenotype - such as neonatal cholestatic jaundice, but this approach is not without limitations either. In 10% of patients only a single pathogenic mutation can be identified, and in some patients new mutations of uncertain clinical significance may be identified.

It has been demonstrated that Hsp70 levels in the brain and liver of a Niemann Pick disease Type C (NPC) mouse model (*Npc1*^{*-*/*-*}) is lower than Hsp70 levels in the wild-type mouse organs (Kirkegaard, T. *et al.* 2016).

Thus, a number of diseases present with a reduced level of Hsp70 in affected tissues, such as the neurons of the CNS and PNS as well as the organs of afflicted individuals.

### Summary

A number of diseases present with reduced level of Hsp70, and it would be highly useful to apply this finding of reduced Hsp70 levels as a general biomarker for aiding in identifying said diseases, or alternatively identifying the subset of patients with diseases co-presenting with a reduced level of Hsp70.

This finding may be applied in methods for diagnosing of patients with a disease presenting with a reduced level of Hsp70, regardless of the pathological consequences and symptoms of such reduced Hsp70, and alternatively identifying or diagnosing the subset of patients with diseases co-presenting with a reduced level of Hsp70. It may also find use in methods for monitoring of disease progression and efficacy of therapy in individuals with a disease presenting with a reduced level of Hsp70.

Thus, in addition to the measurement of Hsp70 levels, a further level of diagnosis is usually performed separately, simultaneously or subsequently in order to determine the specific disease presenting or co-presenting with a reduced level of Hsp70. Correct diagnosis is pivotal for enabling the correct treatment regimens, including those treatments which are approved and specific to the given pathology and opening for the possibility for testing combination treatment with therapies of Hsp70 supplementing and induction.

A reduced level of Hsp70 has been demonstrated in brain tissue and liver tissue samples from a Niemann Pick disease Type C mouse model. However, a biomarker found in tissues such as brain tissue and liver tissue samples is not considered applicable in terms of diagnosing of NPC, let alone any other disease presenting with a reduced level of Hsp70. Hence, other means for identifying diseases presenting with a reduced level of Hsp70 are warranted.

The present inventors have surprisingly found that specifically peripheral blood mononuclear cell (PBMC) samples from patients with Niemann Pick disease Type C contain substantially reduced or decreased levels of Heat Shock Protein 70 (Hsp70), as compared to healthy controls.

A PBMC sample can be easily obtained from an individual by simply drawing a blood sample, and performing a few simple steps of separation and isolation of PBMCs. Hence, the new observation that the low Hsp70 levels observed in pathologically afflicted disease tissue, including brain and liver tissue, directly translate into low levels of Hsp70 protein in PBMC samples, provides a simple, easy and facile detection means of Hsp70 levels.

It is an aspect of the present disclosure to provide a method of detecting Hsp70 in a peripheral blood mononuclear cell (PBMC) sample, said method comprising the steps of
a) providing a PBMC sample, and
b) detecting Hsp70 in said PBMC sample, and
c) optionally quantifying or determining the level of Hsp70 in said PBMC sample.

In a further aspect of the present disclosure there is provided a method for diagnosing a disease presenting with a reduced level of Hsp70 in an individual, said method comprising the steps of:
a) providing a PBMC sample from said individual,
b) detecting Hsp70 in said PBMC sample,
c) quantifying or determining the level of Hsp70 in said PBMC sample, and
d) optionally, classifying or determining whether or not the individual has, or is likely to have, a disease presenting with a reduced level of Hsp70.

In a further aspect of the present disclosure there is provided a method for selecting a patient having a disease presenting with a reduced level of Hsp70, said method comprising the steps of
a) providing a PBMC sample from said patient,
b) detecting Hsp70 in said PBMC sample,
c) quantifying or determining the level of Hsp70 in said PBMC sample, and
d) classifying or determining whether or not the individual has reduced levels of Hsp70.

In a further aspect of the present disclosure there is provided a method for monitoring disease progression in an individual having a disease presenting with a reduced level of Hsp70, said method comprising the steps of
i. providing one or more PBMC samples from said individual at two or more subsequent points in time,
ii. detecting Hsp70 in each of said PBMC samples,
iii. quantifying or determining the level of Hsp70 in each of said PBMC samples.

In a further aspect of the present disclosure there is provided a method for monitoring efficacy of a therapy for treatment of a disease presenting with a reduced level of Hsp70 in an individual having a disease presenting with a reduced level of Hsp70, said method comprising the steps of
a) providing one or more PBMC samples from said individual before, during and/or after a therapy has been applied, maintained, reduced or elevated,
b) detecting Hsp70 in each of said one or more PBMC samples,
c) quantifying or determining the level of Hsp70 in each of said one or more PBMC samples.

In one embodiment said disease presenting with a reduced level of Hsp70 is selected from the group consisting of a lysosomal storage disorder, a neurodegenerative disease, a neuromuscular disorder, muscular dystrophy and an inflammatory muscle disorder.

### Description of Drawings

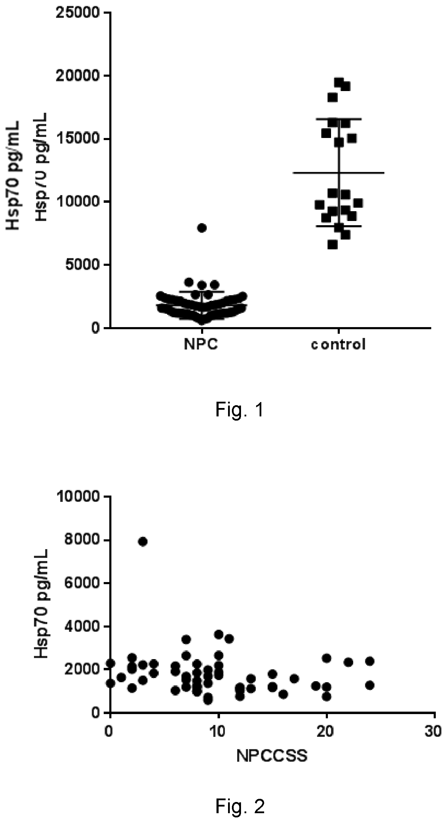

Figure 1. Quantification of the level of Hsp70 protein in PBMC samples isolated from individuals with Niemann Pick disease Type C (NPC) at a first and at a second clinical study visit, or from healthy individuals (controls). The level of Hsp70 was markedly reduced in PBMC samples obtained from NPC patients as compared with PBMC samples obtained from healthy controls, as determined by ELISA.
Figure 2.
   Comparison of the NPC-severity scale score (NPCCSS) with the Hsp70 level in PBMC samples obtained from individuals with Niemann Pick disease Type C. No correlation between Hsp70 level and NPCCSS was observed.
Figure 3.
   Comparison of the Hsp70 level in PBMC samples obtained from individuals with Niemann Pick disease Type C at a first and a second visit. The second clinical study visit was performed 6 to 14 months following the first clinical study visit. No change in Hsp70 level was observed over the time from clinical study visit 1 and 2.
Figure 4.
   HSP70 levels in NPC patients treated with arimoclomol incl. pretreatment.
Figure 5.
   HSP70 levels in NPC patients treated with arimoclomol.

### Detailed description

The present invention is based on the finding of decreased levels of Hsp70 in peripheral blood mononuclear cells (PBMC) samples from patients with Niemann Pick disease Type C, a lysosomal storage disease presenting with reduced levels of Hsp70 in pathologically afflicted disease tissue, including brain and liver tissue, as compared to a control.

Hsp70 proteins are involved in a wide range of cellular processes including protein folding and degradation of unstable cellular proteins as well as serving other cytoprotective roles. Thus, Hsp70 serves several important roles in cell homeostasis, and a number of diseases present with a reduced level of Hsp70, including lysosomal storage diseases, neurodegenerative diseases, and some neuromuscular and muscular diseases.

LSD are a group of rare inherited metabolic disorders that result from defects in lysosomal function as a consequence of deficiency of a single lysosomal protein or enzyme required for the metabolism or transport of lipids, glycolipids, glycoproteins or mucopolysaccharides. Although each disorder results from different gene mutations that translate into a deficiency in protein activity, they all share a common biochemical characteristic - all lysosomal disorders originate from an abnormal accumulation of substances inside the lysosome. Lysosomal storage diseases affect mostly children and they often die at a young and unpredictable age, many within a few months or years of birth. Many other children die of this disease following years of suffering from various symptoms of their particular disorder.

Niemann Pick disease Type C (NPC) is a devastating lysosomal storage disease of the sphingolipidosis-type caused by mutations in either the *NPC1* or *NPC2* gene, resulting in a dysfunctional lysosomal compartment and aberrant accumulation of cholesterol, sphingosine and glycosphingolipids in multiple tissues. Identification of biomarkers to follow disease progression in blood samples has mainly been focused on cholesterol and its derivatives.

NPC also present with reduced levels of Hsp70 in brain and liver tissue, and now the inventors have identified that this finding translates into reduced levels of Hsp70 in peripheral blood mononuclear cells (PBMC) samples from NPC patients.

Patients with diseases presenting with a reduced level of Hsp70 may benefit from increasing the level of Hsp70. Thus, the present invention in one embodiment provides means for identifying patients with a disease presenting with a reduced level of Hsp70, which individual may benefit from increasing the level of Hsp70.

In one embodiment increasing the level of Hsp70 comprises increasing the intracellular concentration of Hsp70 by amplifying Hsp70 gene expression.

In one embodiment increasing the level of Hsp70 comprises increasing the intracellular concentration of Hsp70 by administering Hsp70, or a functional fragment or variant thereof.

### The Heat Shock Protein 70 Family

Hsp70 proteins are involved in a wide range of cellular processes including protein folding and degradation of unstable cellular proteins as well as serving other cytoprotective roles. The common function of Hsp70 in these processes appears to be the binding of short hydrophobic segments in partially folded polypeptides, thereby facilitating proper folding and preventing aggregation. In eukaryotes, Hsp70 chaperones interact *in vivo* with different classes of proteins that serve to regulate critical steps of their functional cycle; amongst these the J-domain family protein Hsp40. Furthermore, additional partner proteins have been identified, some of which are linking Hsp70 to other chaperone systems such as the Hsp90 system.

### Members of the Human Hsp70 Family

Some of the important functions attributed to the molecular chaperones include import of proteins into cellular compartments, folding of proteins in the cytosol, endoplasmic reticulum and mitochondria, prevention of protein aggregation and refolding of misfolded proteins. At present the human Hsp70 family includes 15 members encoded by different genes. The Hsp70 genes and proteins may be referred to herein by their locus name. Reference to Hsp70 usually refers to the two major inducible Hsp70 family members with loci names HSPA1A and HSPA1B, but may also refer to the whole Hsp70 family in general as evident from the consensus of the text.

### HspA1A and HspA1B

The genes transcribed from the loci HSPA1A and HSPA1B are the two heat/stress-inducible Hsp70-genes and the majority of the literature concerning human Hsp70 refers to the proteins encoded by these two genes. The genes give rise to proteins consisting of 641 amino acids, having 99% homology to each other and were the first human Hsp70 family members to be cloned and characterized. The genes are linked in the MHC-class III complex at 6p21.3, are intron-less and with promoter regions containing HSEs, enabling them to bind HSFs and induce transcription in response to a variety of cellular assaults.

### HspA1L and HspA2

Two Hsp70 family members have been termed "chauvinist genes" because male germ cells favor their expression with strong prejudice. The *hspA1L* gene is a constitutively expressed intron-less Hsp70 family member located 4kb telomeric to the HSPA1A locus in the same MHC-class III complex on chromosome 6. It is expressed in low amounts both before and after heat shock but with the expression pattern favoring the testes in mouse, rat and humans with the 641 amino acids (aa) protein being 90% homologous to HspA1A. The *hspA2* gene was first isolated from a mouse genomic library and has later been shown to be constitutively expressed albeit in low levels in various tissues in the human body including skeletal muscle, ovary, small intestine, colon, brain, placenta and the kidneys, but highly expressed in testis. Its expression, or rather lack thereof, has been connected with abnormal human spermatogenesis and male *hspA2*^{*(-*/*-)*} mice are sterile. The gene is located on chromosome 14, giving rise to a 639 aa protein with 84% homology to HspA1A, although the exact location is subject to discussion as two papers have presented different loci positions - 14q24.1 vs. 14q22.

### HspA6 and HspA7

The *hspA6* and *hspA7* genes are heat inducible members of the Hsp70 family with no apparent counterparts in mice. They contain HSEs in their promoter-sites and the genes are intron-less. They are co-localized on chromosome 1 and are 94% homologous to each other in the nucleotide sequence. However, only HspA6 is functional as the *hspA7* gene harbors a single nucleotide insertion generating a premature stop codon at +1324. The HspA6 protein is 643 aa long and shows 77% homology to HspA1A and HspA1B.

### HspA5 and HspA9

The *hspA5* and *hspA9* genes are the two compartment-specific members of the Hsp70 family. The 655 aa HspA5 protein is located in the endoplasmic reticulum (ER) and facilitates folding and transport of newly synthesized proteins in this compartment. The protein is 64% homologous to HspA1A, the gene being located at 9q34. The 679 aa HspA9 protein is located in the mitochondria where it assists in folding of proteins after their transport across the mitochondrial membrane. HspA9 is located at 5q31.1, the protein being 52% homologous to HspA1A.

### HspA8

The cognate Hsp70 member known as Hsc70 is encoded by a gene named *hspA8* at 11q24, giving rise to a 646 aa protein with 86% homology to HspA1A, and is constitutively expressed in all tissues and cell lines. The protein is analogous to Hsp70 in its cellular functions, providing the required chaperoning under normal circumstances, but has also been ascribed a role in the un-coating of clathrin-coated vesicles as well as in chaperone-mediated autophagy. HspA3 and HspA4, HspA4L, HspA12A and HspA14 will not be further discussed herein.

**Table 1: List of the Human Hsp70 Gene Family. The genes are listed according to locus name, names used herein, chromosomal location (position), amino acid homology to HspA1A as well as alternative names often seen in the literature.**

| **Locus** | **Name Used herein, Gene/Protein** | **Position** | **% aa Homology to HSPA1A** | **Alternative Names** |
|---|---|---|---|---|
| HSPA1A | *hspA1A*/HspA1A (Hsp70) | 6p23.1 | 100 | Hsp70; Hsp72; Hsp70-1 |
| HSPA1B | *hspA1B*/HspA1B (Hsp70) | 6p23.1 | 99 | Hsp70; Hsp72; Hsp70-2 |
| HSPA1L | *hspA1L*/HspA1L | 6p23.1 | 90 | Hsp70-Hom; Hsp70t |
| HSPA2 | *hspA2*/HspA2 | 14q24.1 | 84 | Hsp70-3 |
| HSPA4 | *hspA4*/HspA4 | 5q31.1 | 31 | Hsp70RY; APG-2 |
| HSPA4L | *hspA4L*/*HspA4L* | | | APG1; OSP94 |
| HSPA5 | *hspA5*/HspA5 | 9q34 | 64 | BiP; GRP78 |
| HSPA6 | *hspA6*/HspA6 | 1q | 84 | Hsp70-6; Hsp70B' |
| HSPA7 | *hspA7*/HspA7 | 1q | - | Hsp70-7; Hsp70B |
| HSPA8 | *hspA8*/HspA8 (Hsc70) | 11q24 | 86 | Hsc70; Hsp73 |
| HSPA9 | *hspA9*/HspA9 | 5q31.1 | 52 | GRP75; PBP74; mtHsp75; mortalin; mot-2 |
| HSPA12A | *hspA12A*/*HspA12A* | | | KIAA0417 |
| HSPA14 | *hspA14*/*HspA14* | | | Hsp60; Hsp70L1 |

### Methods involving Hsp70 biomarker

The inventors have found that Hsp70 levels are significantly lower in specifically PBMC samples obtained from patients with a disease known to present with a reduced level of Hsp70 in pathologically afflicted disease tissue, including brain and liver tissue, as compared to healthy controls. Diseases presenting with a reduced level of Hsp70 may be alleviated by Hsp70 therapies and the present disclosure thus provides means for selecting patients which may benefit from treatment with Hsp70 therapies.

'Reduced' levels may be used interchangeably with decreased or lower levels herein. The Hsp70 levels may be reduced or lower as compared to a control, such as a healthy individual, or as compared to an different time point (e.g. first visit/sample compared to a later visit/sample).

The present methods allow for easy and facile detection of said Hsp70 levels in PBMC samples and enable the use of Hsp70 in PBMCs as a biomarker to provide a reliable and easy tool to identify diseases presenting with a reduced level of Hsp70.

The present methods comprise the detection and quantification of Hsp70 in PBMC sample obtained from an individual.

Detection and quantification of Hsp70 in PBMC samples can thus be used in methods of diagnosing or identifying a patient with a disease presenting with a reduced level of Hsp70.

Accordingly, a reduced amount of Hsp70 in a PBMC sample from an individual as compared to the amount detected in healthy controls is indicative of a disease presenting with a reduced level of Hsp70; or indicative of the patient being eligible for Hsp70 therapies.

The present methods can also be used in methods of monitoring disease progression in an individual or patient with a disease presenting with a reduced level of Hsp70.

The present methods can also be used in methods of monitoring efficacy of a treatment in an individual or patient with a disease presenting with a reduced level of Hsp70.

### Methods of detecting Hsp70

It is an aspect of the present disclosure to provide a method of detecting Hsp70 in a PBMC sample, said method comprising the steps of
a) providing a PBMC sample,
b) detecting Hsp70 in said PBMC sample, and
c) optionally quantifying or determining the level of Hsp70 in said PBMC sample.

In one embodiment, detecting and/or quantifying Hsp70 refer to the detection and quantification of Hsp70 protein.

In one embodiment, detecting and/or quantifying Hsp70 refer to the detection and quantification of one or both of HspA1A and HspA1B.

In one embodiment, detecting and/or quantifying Hsp70 refer to the detection and quantification of one or both of HspA1A and HspA1B, with no or little detection of HspA5 and/or HspA8.

In one embodiment, detecting and/or quantifying Hsp70 includes the detection of naturally occurring Hsp70 and naturally occurring Hsp70 variants, such as naturally occurring HspA1A and/or HspA1B and naturally occurring HspA1A and/or HspA1B variants. Naturally occurring HspA1A and/or HspA1B variants are known to the skilled person.

In one embodiment said Hsp70 is selected from HspA1A (SEQ ID NOs: 1 and 2) and HspA1B (SEQ ID NOs: 4 and 5), or a functional fragment or variant thereof. In SEQ ID NO: 2 the initiator methionine (M at position 1) of SEQ ID NO: 1 is removed. In SEQ ID NO: 5 the initiator methionine (M at position 1) of SEQ ID NO: 4 is removed. *In vivo* this occurs by post-translational processing.

In one embodiment said method of detecting Hsp70 in a PBMC sample is an *in vitro* method.

Quantifying, in one embodiment, means determining the level of said Hsp70 protein present in the PBMC sample.

In one embodiment the level of Hsp70 is detected and/or quantified in a PBMC sample. In one embodiment said PBMC sample is obtained from or obtainable from an individual. In one embodiment said individual has, is suspected of having, is at risk of having or is likely to have, a disease presenting with a reduced level of Hsp70. In one embodiment said individual has, is suspected of having, is at risk of having or is likely to have, a lysosomal storage disease selected from the group consisting of lipid storage disorders including the sphingolipidoses; mucopolysaccharidoses; glycogen storage disorders; disorders of glycoprotein metabolism (glycoproteinosis); and mucolipidoses, and any subtype thereof as specified herein elsewhere (including i.a. Niemann Pick disease). In one embodiment said individual has, is suspected of having, is at risk of having or is likely to have, a neurodegenerative disease, a neuromuscular disorder, muscular dystrophy or an inflammatory muscle disorder, as specified herein elsewhere. The PBMC samples are disclosed in further detail herein elsewhere.

In one embodiment the present disclosure thus provides a method of detecting Hsp70 in a PBMC sample comprising the steps of
a) providing a PBMC sample,
b) detecting Hsp70 in said PBMC sample, and
c) quantifying or determining the level of Hsp70 in said PBMC sample.

Also disclosed is a method of detecting HspA1A and/or HspA1B in a PBMC sample, said method comprising the steps of
a) providing a PBMC sample,
b) detecting HspA1A and/or HspA1B in said PBMC sample, and
c) optionally quantifying or determining the level of HspA1A and/or HspA1B in said PBMC sample.

In one embodiment said methods for detecting and optionally quantifying Hsp70 in a PBMC sample comprise detecting and optionally quantifying:
i) HspA1A,
ii) HspA1B, or
iii) HspA1A and HspA1B.

The present methods of detecting and optionally quantifying HspA1A and/or HspA1B in one embodiment do not exclude the detection of other Hsp70 proteins.

In one embodiment the present disclosure thus provides a method of detecting Hsp70 in a PBMC sample comprising the steps of
a) providing a PBMC sample,
b) detecting Hsp70 in said PBMC sample, and
c) quantifying or determining the level of Hsp70 in said PBMC sample,
wherein said PBMC sample is obtained from an individual having, suspected of having, at risk of having or likely to have a disease presenting with reduced levels of Hsp70, including a lysosomal storage disease, a neurodegenerative disease, a neuromuscular disorder, muscular dystrophy or an inflammatory muscle disorder as specified herein elsewhere.

Also disclosed herein is a method of detecting and optionally quantifying Hsp70 in a PBMC sample from an individual or patient with a disease presenting with a reduced level of Hsp70 before, during and/or after a therapy has been applied, maintained, reduced or elevated.

In one embodiment said individual has a lysosomal storage disease selected from the group consisting of lipid storage disorders including the sphingolipidoses; mucopolysaccharidoses; glycogen storage disorders; disorders of glycoprotein metabolism (glycoproteinosis); and mucolipidoses, and any subtype thereof as specified herein elsewhere (including i.a. Niemann Pick disease). In one embodiment said individual has a neurodegenerative disease, a neuromuscular disorder, muscular dystrophy or an inflammatory muscle disorder, as specified herein elsewhere.

In one embodiment, said therapy comprises a therapy for inducing Hsp70 level and/or activity, such as or a bioactive agent capable of inducing the expression of Hsp70 and/or activity of Hsp70. In one embodiment, said therapy comprises a therapy for treatment of a disease presenting with a reduced level of Hsp70, such as a bioactive agent effective in the treatment of a disease presenting with a reduced level of Hsp70. Therapies and bioactive agents are disclosed herein elsewhere.

In one embodiment said method of detecting and optionally quantifying Hsp70 in a PBMC sample from an individual comprises
i) one or more PBMC samples obtained from an individual having a disease presenting with a reduced level of Hsp70 before a therapy has been applied, maintained, reduced or elevated;
ii) one or more PBMC samples obtained from an individual having a disease presenting with a reduced level of Hsp70 during a therapy; and/or
iii) one or more PBMC samples obtained from an individual having a disease presenting with a reduced level of Hsp70 after a therapy has been applied, maintained, reduced or elevated; and/or;
iv) one or more PBMC samples obtained from an individual having a disease presenting with a reduced level of Hsp70 before and after a therapy has been applied, maintained, reduced or elevated.

Also disclosed herein is a method of detecting and optionally quantifying Hsp70 in a PBMC sample from an individual having a disease presenting with a reduced level of Hsp70 at subsequent points in time to monitor disease progression.

Also disclosed herein is a method of detecting and optionally quantifying Hsp70 in a PBMC sample from an individual suspected of having a disease presenting with a reduced level of Hsp70, to diagnose said disorder.

Means for providing a PBMC sample, the nature of the PBMC sample and means for detection of Hsp70 and for quantifying Hsp70 are disclosed herein elsewhere.

### Detection and quantification of Hsp70

The methods disclosed herein comprise one or more steps of detecting Hsp70 in a PBMC sample, including the steps of
a) providing one or more PBMC samples from an individual,
b) detecting Hsp70 in said PBMC sample, and
c) optionally quantifying or determining the level of Hsp70 in said PBMC sample.

Hsp70 in PBMC according to the present disclosure may be detected at the DNA level, the RNA/mRNA level and/or the protein level. In a preferred embodiment, Hsp70 is detected at the protein level.

Detection and quantification of Hsp70 can be performed by several methods known in the art, and may include one or multiple steps. Detection and quantification of Hsp70 may be performed by any method known to the skilled person.

In one embodiment the steps of b) detecting Hsp70 in said PBMC sample is performed by subjecting the PBMC sample to one or more steps of detection and/or quantification.

In one embodiment, detection and quantification of Hsp70 protein is performed by a Spectrometry methods or an Antibody dependent method.

In one embodiment, detection and quantification of Hsp70 protein is performed by a method selected from the group consisting of enzyme-linked immunosorbent assay (ELISA), western blotting, Protein immunoprecipitation, Immunoelectrophoresis, Protein immunostaining, High-performance liquid chromatography (HPLC) and Liquid chromatography-mass spectrometry (LC/MS).

In one embodiment, detection and quantification of Hsp70 protein is performed using mass spectrometry. In one embodiment, detection and quantification of Hsp70 protein in a dried blood spot (DBS) sample is performed using mass spectrometry.

Dried blood spot testing (DBS) is a form of biosampling, well-known to a person of skill in the art, where blood samples, such as 70µL of whole blood per spot, are blotted and dried on filter paper. The dried samples can easily be shipped to an analytical laboratory and analyzed using various methods, such as the methods mentioned herein, for example mass spectrometry. In one embodiment, the total protein is extracted from the blood spots soaked in phosphate buffered saline (PBS). In one embodiment, the protein is further subjected to trypsin digest, purified and analyzed by mass spectrometry.

In one embodiment, detection and quantification of Hsp70 protein is performed by means of enzyme-linked immunosorbent assay (ELISA).

In one embodiment the step of b) detecting Hsp70 in said PBMC sample and/or the step c) optionally quantifying or determining the level of Hsp70 in said PBMC sample, comprises one or more steps of
i) lysis of the PBMC sample, and/or
ii) immobilization of Hsp70 protein present in the lysed PBMC sample, and/or
iii) binding of primary antibodies to said immobilized Hsp70 protein, and/or
iv) binding of secondary antibodies to the primary antibody of iii), and/or
v) binding of streptavidin conjugates to the secondary antibody, to provide a streptavidin conjugate, and/or
vi) visualization of the primary antibody bound to Hsp70 protein, and/or
vii) visualization of the secondary antibody.

In one embodiment, the primary antibody of iii) is covalently associated with a biotin molecule to allow binding of the streptavidin conjugate of said secondary antibody.

In one embodiment, said primary antibody, said secondary antibody or said streptavidin conjugate of v) is covalently associated with an enzyme or a fluorescent probe.

Step vi) and vii) visualization of the primary antibody bound to Hsp70 or the secondary antibody bound to the primary antibody may be performed by colorimetric detection, chemiluminescent detection, radioactive detection, electrochemical detection or fluorescent detection. In one embodiment the visualization comprises conversion of one or more substrates by an enzyme covalently associated with said primary antibody bound to Hsp70, said secondary antibody bound to the primary antibody or said streptavidin conjugate bound to the primary antibody.

In one embodiment, the enzyme covalently associated with said primary antibody bound to Hsp70, said secondary antibody bound to the primary antibody or said streptavidin conjugate is horse radish peroxidase (HRP). In one embodiment, said substrate is selected from the group consisting of hydrogenperoxide, luminol, tetramethylbenzidine.

In one embodiment, the step of b) detecting Hsp70 in said PBMC sample and/or the step c) optionally quantifying or determining the level of Hsp70 in said PBMC sample further comprises washing steps in between the steps of lysis, immobilization, binding, and visualization.

ELISA typically is suitable to measure presence and/or amount of a given protein in a sample. Hence ELISA is suitable for measuring the presence and/or amount of Hsp70 in a PBMC sample.

In one embodiment the step of b) detecting Hsp70 in said PBMC sample and/or the step c) optionally quantifying or determining the level of Hsp70 in said PBMC sample, comprises one or more steps of detecting Hsp70 directly (e.g. by ELISA).

In one embodiment, a calibrated standard is used for quantification of Hsp70 present in the PBMC sample.

In one embodiment the calibrated standard for quantification is used at different concentrations.

In one embodiment, detection and quantification of Hsp70 mRNA is performed by a method selected from the group consisting of Northern blot, ribonuclease protection assay (RPA), and real-time polymerase chain reaction (RT-PCR).

### Methods of diagnosing

It is an aspect of the present disclosure to provide a method for diagnosing a disease presenting with a reduced level of Hsp70 in an individual, said method comprising the step of detecting Hsp70 in a PBMC sample obtained from or obtainable from an individual.

In one embodiment said method for diagnosing a disease presenting with a reduced level of Hsp70 further comprises the step of quantifying or determining the level of Hsp70 present in the PBMC sample.

In one embodiment the level of the Hsp70 present in the PBMC sample is indicative of whether or not the individual has (or is suffering from) a disease presenting with a reduced level of Hsp70, or whether or not the individual is likely to have or at risk of having (or suffering from) a disease presenting with a reduced level of Hsp70.

The methods may be used to confirm a suspected diagnosis, for example if there is a suspicion that the individual has or suffers from a disease presenting with a reduced level of Hsp70, based e.g. on family history, or on the presence of symptoms indicative of an LSD. The methods may be used in addition to known methods of diagnosing a disease presenting with a reduced level of Hsp70.

It is thus an aspect of the present disclosure to provide a method for diagnosing a disease presenting with a reduced level of Hsp70 in an individual, said method comprising the steps of
a) providing a PBMC sample from said individual,
b) detecting Hsp70 in said PBMC sample, and
c) quantifying or determining the level of Hsp70 in said PBMC sample.

In one embodiment the level of the Hsp70 present in the PBMC sample is indicative of whether or not the individual has (or is suffering from) a disease presenting with a reduced level of Hsp70, or whether or not the individual is likely to have or at risk of having (or suffering from) a disease presenting with a reduced level of Hsp70.

Also disclosed is a method for diagnosing a disease presenting with a reduced level of Hsp70 in an individual, said method comprising the steps of
a) providing a PBMC sample from said individual,
b) detecting Hsp70 in said PBMC sample,
c) quantifying or determining the level of Hsp70 in said PBMC sample, and
d) classifying or determining whether or not the individual has, or is likely to have, a disease presenting with a reduced level of Hsp70.

In one embodiment said PBMC sample is obtained from or obtainable from an individual.

In one embodiment there is disclosed a method for diagnosing a disease presenting with a reduced level of Hsp70 in an individual, said method comprising the steps of
a) providing a PBMC sample from said individual,
b) detecting Hsp70 in said PBMC sample,
c) quantifying or determining the level of Hsp70 in said PBMC sample, and
d) classifying or determining whether or not the individual has, or is likely to have, a disease presenting with a reduced level of Hsp70,
wherein said disease is a lysosomal storage disease, a neurodegenerative disease, a neuromuscular disorder, muscular dystrophy or an inflammatory muscle disorder as specified herein elsewhere.

In one embodiment said lysosomal storage disease is selected from the group consisting of lipid storage disorders including the sphingolipidoses; mucopolysaccharidoses; glycogen storage disorders; disorders of glycoprotein metabolism (glycoproteinosis); and mucolipidoses, and any subtype thereof as specified herein elsewhere. In one embodiment said lysosomal storage disease is Niemann Pick disease, such as NPC.

In one embodiment said method for diagnosing a disease presenting with a reduced level of Hsp70 comprise detecting and quantifying or determining the level of
i) HspA1A,
ii) HspA1B, or
iii) HspA1A and HspA1B.

In one embodiment, step d) classifying or determining whether or not the individual has, or is likely to have, a disease presenting with a reduced level of Hsp70, is a step of classifying or determining the individual as having, or likely to have a disease presenting with a reduced level of Hsp70.

Reference to 'the sample' or 'a sample' herein will refer to a PBMC sample from the individual having or suspected of having a disease presenting with a reduced level of Hsp70, unless otherwise specified. In contrast, a sample from a healthy control will be referred to as a PBMC sample from a healthy control or control sample.

In one embodiment said step d) of classifying or determining the individual as having, or likely to have, a disease presenting with a reduced level of Hsp70, comprises determining the level of Hsp70 in the PBMC sample as compared to the levels in a PBMC sample obtained or obtainable from a healthy control. A healthy control in the present context is an individual who does not have, or is not suspected of having, a disease presenting with a reduced level of Hsp70. Preferably the healthy control also does not present with any other apparent disease. In one embodiment, the healthy control can be of any age. In one embodiment, the healthy control is an age-matched control. In one embodiment, the healthy control is below the age of 30, such as below 29, such as below 28, such as below 27, such as below 26, such as below 25, such as below 24, such as below 23, such as below 22, such as below 21, such as below 20, such as below 19, such as below the age of 18. In one embodiment, the healthy control is below the age of 20. In one embodiment, the healthy control is of age from about 4 to about 18. In one embodiment, the healthy control is of age from about 2 to about 4.

In one embodiment a decreased level of Hsp70 in the PBMC sample as compared to levels in a healthy control is indicative of the individual having, likely to have or at risk of having, a disease presenting with a reduced level of Hsp70.

In one embodiment a level of Hsp70 in the PBMC sample which is comparable to or equal to the level in a healthy control is indicative of the individual not having a disease presenting with a reduced level of Hsp70.

In one embodiment a level of Hsp70 in the PBMC sample which is higher than the level in a healthy control is indicative of the individual not having a disease presenting with a reduced level of Hsp70.

In one embodiment the individual is likely to have a disease presenting with a reduced level of Hsp70 if
i) the level of Hsp70 in the PBMC sample is lower than the level found in healthy controls, or undetectable,
ii) the level of HspA1A in the PBMC sample is lower than the level found in healthy controls, or undetectable,
iii) the level of HspA1B in the PBMC sample is lower than the level found in healthy controls, or undetectable, and/or
iv) the level of HspA1A and HspA1B in the PBMC sample is lower than the level found in healthy controls, or undetectable

In one embodiment the individual is likely to have a disease presenting with a reduced level of Hsp70 if the level of Hsp70 in the PBMC sample is 1 to 1000 times lower than the level found in healthy controls, such as 1 to 2 times, 2 to 3 times, 3 to 4 times, 4 to 5 times, 5 to 6 times, 6 to 7 times, 7 to 8 times, 8 to 9 times, 9 to 10 times, 10 to 11 times, 11 to 12 times, 12 to 13 times, 13 to 14 times, 14 to 15 times, 15 to 16 times, 16 to 17 times, 17 to 18 times, 18 to 19 times, 19 to 20 times, 20 to 25 times, 25 to 30 times, 30 to 35 times, 35 to 40 times, 40 to 45 times, 45 to 50 times, 50 to 75 times, 75 to 100 times, 100 to 150 times, 150 to 200 times, 200 to 250 times, 250 to 300 times, 300 to 400 times, 400 to 500 times, 500 to 750 times, 750 to 1000 times lower than the level found in a healthy control, or undetectable.

In one embodiment the individual is likely to have a disease presenting with a reduced level of Hsp70 if the level of HspA1A and/or HspA1B in the PBMC sample is 1 to 1000 times lower than the level found in healthy controls, such as 1 to 2 times, 2 to 3 times, 3 to 4 times, 4 to 5 times, 5 to 6 times, 6 to 7 times, 7 to 8 times, 8 to 9 times, 9 to 10 times, 10 to 11 times, 11 to 12 times, 12 to 13 times, 13 to 14 times, 14 to 15 times, 15 to 16 times, 16 to 17 times, 17 to 18 times, 18 to 19 times, 19 to 20 times, 20 to 25 times, 25 to 30 times, 30 to 35 times, 35 to 40 times, 40 to 45 times, 45 to 50 times, 50 to 75 times, 75 to 100 times, 100 to 150 times, 150 to 200 times, 200 to 250 times, 250 to 300 times, 300 to 400 times, 400 to 500 times, 500 to 750 times, 750 to 1000 times lower than the level found in a healthy control, or undetectable.

In one embodiment 'undetectable' means that no signal is observed, such as no signal above the baseline noise. In one embodiment 'undetectable' means that the level of Hsp70 is undetectable in the PBMC sample.

In one embodiment said step d) of classifying or determining the individual as having, or likely to have, a disease presenting with a reduced level of Hsp70, comprises determining if the amount of Hsp70 in said PBMC sample is below a predefined cut-off value, or undetectable.

If the amount of Hsp70 is below said cut-off value, the individual has or is likely to have a disease presenting with a reduced level of Hsp70. If the amount of Hsp70 is equal to or above said cut-off value, the individual does not have or is not likely to have a disease presenting with a reduced level of Hsp70.

In one embodiment said step d) of classifying or determining the individual as *not* having, or not likely to have, a disease presenting with a reduced level of Hsp70, comprises determining if the amount of Hsp70 in said PBMC sample is equal to or above a predefined cut-off value.

Said cut-off values are determined based on the value in healthy controls, and compared to the value in individuals with a disease presenting with a reduced level of Hsp70.

In one embodiment said cut-off values are determined based on the value in healthy controls compared to the value in patients with a LSD, such as Niemann Pick disease, such as Niemann Pick disease Type C.

A cut-off value for Hsp70 of 5000 pg/mL PBMC means that a value of 5000 pg/mL Hsp70 or less is indicative of the individual having, or likely to have, a disease presenting with a reduced level of Hsp70.

In one embodiment the individual has or is likely to have a disease presenting with a reduced level of Hsp70 if the amount of Hsp70 in said PBMC sample is 7500 pg/mL or less, such as 7000 pg/mL or less, such as 6500 pg/mL or less, such as 6000 pg/mL or less, such as 5500 pg/mL or less, such as 5000 pg/mL or less, such as 4500 pg/mL or less, such as 4000 pg/mL or less, such as 3500 pg/mL or less, such as 3000 pg/mL or less, such as 2500 pg/mL or less, such as 2000 pg/mL or less, such as 1500 pg/mL or less, such as 1000 pg/mL PBMC or less.

In one embodiment the individual has or is likely to have a disease presenting with a reduced level of Hsp70 if the amount of HspA1A and/or HspA1B in said PBMC sample is 7500 pg/mL or less, such as 7000 pg/mL or less, such as 6500 pg/mL or less, such as 6000 pg/mL or less, such as 5500 pg/mL or less, such as 5000 pg/mL or less, such as 4500 pg/mL or less, such as 4000 pg/mL or less, such as 3500 pg/mL or less, such as 3000 pg/mL or less, such as 2500 pg/mL or less, such as 2000 pg/mL or less, such as 1500 pg/mL or less, such as 1000 pg/mL PBMC or less.

Conversely, an individual is classified as not having or not likely to have a disease presenting with a reduced level of Hsp70 if the amount of Hsp70 in said PBMC sample is above 5000 pg/mL, such as above 5500 pg/mL, such as above 6000 pg/mL, such as above 6500 pg/mL, such as above 7000 pg/mL, such as above 7500 pg/mL, such as above 8000 pg/mL, such as above 8500 pg/mL, such as above 9000 pg/mL, such as above 9500 pg/mL, such as above 10000 pg/mL, such as above 10500 pg/mL, such as above 11000 pg/mL, such as above 11500 pg/mL, such as above 12000 pg/mL, such as above 12500 pg/mL PBMC.

In one embodiment an individual is classified as not having or not likely to have a disease presenting with a reduced level of Hsp70 if the amount of HspA1A and/or HspA1B in said PBMC sample is above 5000 pg/mL, such as above 5500 pg/mL, such as above 6000 pg/mL, such as above 6500 pg/mL, such as above 7000 pg/mL, such as above 7500 pg/mL, such as above 8000 pg/mL, such as above 8500 pg/mL, such as above 9000 pg/mL, such as above 9500 pg/mL, such as above 10000 pg/mL, such as above 10500 pg/mL, such as above 11000 pg/mL, such as above 11500 pg/mL, such as above 12000 pg/mL, such as above 12500 pg/mL PBMC.

Furthermore, an individual is classified as having or likely to have a disease presenting with a reduced level of Hsp70 if the amount of Hsp70 in said PBMC sample is undetectable.

The diagnosis may be confirmed or infirmed using methods otherwise known in the art, or by repeating the diagnosis methods disclosed herein.

In one embodiment the present methods further comprises applying, maintaining, reducing, elevating or not applying a therapy based on whether or not the subject has, or is at risk of having a disease presenting with a reduced level of Hsp70.

In one embodiment the method for diagnosing a disease presenting with a reduced level of Hsp70 in an individual comprises the step of e) administering a therapy for treatment of a disease presenting with a reduced level of Hsp70 to the patient diagnosed with said disease presenting with a reduced level of Hsp70.

### Methods of diagnosing and treating

It is also an aspect of the present disclosure to provide a method for diagnosing and treating a disease presenting with a reduced level of Hsp70 in an individual, said method comprising the steps of
a) providing a PBMC sample from said individual,
b) detecting Hsp70 in said PBMC sample,
c) quantifying or determining the level of Hsp70 in said PBMC sample,
d) classifying or determining whether or not the individual has, or is likely to have disease presenting with a reduced level of Hsp70, and
e) administering a therapy for treatment of said disease presenting with a reduced level of Hsp70 to the individual.

In one embodiment step e) of administering a therapy for treatment of a disease presenting with a reduced level of Hsp70 to the individual comprises administering an effective amount of a bioactive agent to said individual, wherein said bioactive agent is effective for said disease presenting with a reduced level of Hsp70.

It is also an aspect of the present disclosure to provide a method of treating an individual with a disease presenting with a reduced level of Hsp70, said method comprising administering a therapy for treatment of said disease presenting with a reduced level of Hsp70 to said individual, wherein said individual is diagnosed with said disease presenting with a reduced level of Hsp70 by a method comprising the steps of:
a) providing a PBMC sample from said individual,
b) detecting Hsp70 in said PBMC sample,
c) quantifying or determining the level of Hsp70 in said PBMC sample,
d) classifying or determining whether or not the individual has, or is likely to have a disease presenting with a reduced level of Hsp70.

A therapy for treatment of a disease presenting with a reduced level of Hsp70 and a bioactive agent for same purpose are disclosed herein elsewhere and included in the above methods of treatment and therapies for disorders related to a reduced level of Hsp70.

In one embodiment said method for diagnosing and treating a disease presenting with a reduced level of Hsp70 in an individual comprises diagnosing and treating a disease selected from the group consisting of a lysosomal storage disease, a neurodegenerative disease, a neuromuscular disorder, muscular dystrophy or an inflammatory muscle disorder as specified herein elsewhere.

In one embodiment said lysosomal storage disease is selected from the group consisting of lipid storage disorders including the sphingolipidoses; mucopolysaccharidoses; glycogen storage disorders; disorders of glycoprotein metabolism (glycoproteinosis); and mucolipidoses, and any subtype thereof as specified herein elsewhere. In one embodiment said lysosomal storage disease is Niemann Pick disease, such as NPC.

### Methods of selecting a patient

The present methods allow for detection of Hsp70 in a PBMC sample and the diagnosis or identification of individuals having a disease presenting with a reduced level of Hsp70.

A number of diseases present with reduced level of Hsp70 as one of a number of biological, molecular and pathological changes. It would be highly useful to easily be able to identify patients having a disease presenting with a reduced level of Hsp70, or the subset of patients with a disease co-presenting with a reduced level of Hsp70, such as presenting or co-presenting with a significantly or markedly reduced level of Hsp70.

The identification of patients having a disease presenting with a reduced level of Hsp70, or a subset of patients with a disease co-presenting with a reduced level of Hsp70, is applicable for selecting patients who will or is likely to respond to a bioactive agent that increase the intracellular concentration and/or activity of heat shock proteins, including Hsp70.

It is thus an aspect to provide a method for selecting a patient having a disease presenting with a reduced level of Hsp70, said method comprising the steps of
a) providing a PBMC sample from said patient,
b) detecting Hsp70 in said PBMC sample,
c) quantifying or determining the level of Hsp70 in said PBMC sample, and
d) classifying or determining whether or not the patient has reduced levels of Hsp70.

In one embodiment said detecting and quantifying or determining the level of Hsp70 comprises detecting and quantifying or determining the level of
i) HspA1A,
ii) HspA1B, or
iii) HspA1A and HspA1B.

In one embodiment the step of classifying or determining whether or not the patient has reduced levels of Hsp70 comprises classifying or determining whether or not the patient has reduced levels of Hsp70 as compared to a control, as specified herein elsewhere.

In one embodiment the patient has reduced levels of Hsp70 when a decreased or undetectable level of Hsp70, such as HspA1A and/or HspA1A, in the PBMC sample as compared to levels in a healthy control is determined.

In one embodiment the step of classifying or determining whether or not the patient has reduced levels of Hsp70 comprises a step of identifying a patient with reduced levels of Hsp70.

In one embodiment said step d) of classifying or determining whether or not the patient has reduced levels of Hsp70, comprises determining the level of Hsp70 in the PBMC sample as compared to the levels in a PBMC sample obtained or obtainable from a healthy control. A healthy control in the present context is an individual who does not have, or is not suspected of having, a disease presenting with a reduced level of Hsp70. Preferably the healthy control also do not present with any other apparent disease.

In one embodiment said step d) of classifying or determining whether or not the patient has reduced levels of Hsp70, comprises determining the level of Hsp70 in the PBMC sample as compared to the levels in a PBMC sample obtained or obtainable from a patient presenting with the same underlying disease but not having accompanying reduced levels of Hsp70.

It one embodiment there is provided a method for selecting a patient having reduced level of Hsp70, wherein said patient has an underlying disease, said method comprising the steps of
a) providing a PBMC sample from said patient,
b) detecting Hsp70 in said PBMC sample,
c) quantifying or determining the level of Hsp70 in said PBMC sample, and
d) classifying or determining whether or not the patient has reduced levels of Hsp70 as compared to other patients presenting with the same underlying disease.

It one embodiment there is provided a method for selecting or identifying a patient with a disease selected from the group consisting of a lysosomal storage disorder, a neurodegenerative disorder, a neuromuscular disorder, muscular dystrophy and an inflammatory muscle disorder, as having a reduced level of Hsp70, said method comprising the steps of
a) providing a PBMC sample from said patient,
b) detecting Hsp70 in said PBMC sample,
c) quantifying or determining the level of Hsp70 in said PBMC sample, and
d) classifying or determining whether or not the patient has reduced levels of Hsp70.

It one embodiment there is provided a method for selecting or identifying a patient with Niemann Pick disease (such as NPC) as having a reduced level of Hsp70, said method comprising the steps of
a) providing a PBMC sample from said patient,
b) detecting Hsp70 in said PBMC sample,
c) quantifying or determining the level of Hsp70 in said PBMC sample, and
d) classifying or determining whether or not the patient has reduced levels of Hsp70.

In one embodiment the patient has a reduced level of Hsp70 if the level of Hsp70 (in one embodiment HspA1A and/or HspA1B) in the PBMC sample is 1 to 1000 times lower than the level found in healthy controls, such as 1 to 2 times, 2 to 3 times, 3 to 4 times, 4 to 5 times, 5 to 6 times, 6 to 7 times, 7 to 8 times, 8 to 9 times, 9 to 10 times, 10 to 11 times, 11 to 12 times, 12 to 13 times, 13 to 14 times, 14 to 15 times, 15 to 16 times, 16 to 17 times, 17 to 18 times, 18 to 19 times, 19 to 20 times, 20 to 25 times, 25 to 30 times, 30 to 35 times, 35 to 40 times, 40 to 45 times, 45 to 50 times, 50 to 75 times, 75 to 100 times, 100 to 150 times, 150 to 200 times, 200 to 250 times, 250 to 300 times, 300 to 400 times, 400 to 500 times, 500 to 750 times, 750 to 1000 times lower than the level found in a healthy control, or undetectable.

In one embodiment said step d) of classifying or determining whether or not the patient has reduced levels of Hsp70, comprises determining if the amount of Hsp70 (in one embodiment HspA1A and/or HspA1B) in said PBMC sample is below a predefined cut-off value, or undetectable.

If the amount of Hsp70 is below said cut-off value, the patient has a reduced level of Hsp70 and is likely, or more likely, to respond to Hsp70 therapies including bioactive agents that increase the intracellular concentration and/or activity of heat shock proteins, including Hsp70. If the amount of Hsp70 is equal to or above said cut-off value, the patient does not have a reduced level of Hsp70 and is less likely, or not likely, to respond to Hsp70 therapies including bioactive agents that increase the intracellular concentration and/or activity of heat shock proteins, including Hsp70.

In one embodiment the patient presenting with a reduced level of Hsp70 is likely, or more likely, to respond to Hsp70 therapies if the amount of Hsp70 (in one embodiment HspA1A and/or HspA1B) in said PBMC sample is 7500 pg/mL or less, such as 7000 pg/mL or less, such as 6500 pg/mL or less, such as 6000 pg/mL or less, such as 5500 pg/mL or less, such as 5000 pg/mL or less, such as 4500 pg/mL or less, such as 4000 pg/mL or less, such as 3500 pg/mL or less, such as 3000 pg/mL or less, such as 2500 pg/mL or less, such as 2000 pg/mL or less, such as 1500 pg/mL or less, such as 1000 pg/mL PBMC or less.

Conversely, in one embodiment the patient is less likely, or not likely, to respond to Hsp70 therapies if the amount of Hsp70 (in one embodiment HspA1A and/or HspA1B) in said PBMC sample is above 5000 pg/mL, such as above 5500 pg/mL, such as above 6000 pg/mL, such as above 6500 pg/mL, such as above 7000 pg/mL, such as above 7500 pg/mL, such as above 8000 pg/mL, such as above 8500 pg/mL, such as above 9000 pg/mL, such as above 9500 pg/mL, such as above 10000 pg/mL, such as above 10500 pg/mL, such as above 11000 pg/mL, such as above 11500 pg/mL, such as above 12000 pg/mL, such as above 12500 pg/mL PBMC.

In one embodiment the step of identifying a patient with reduced levels of Hsp70 comprises a step of determining eligibility of said patient for administering a therapy for treatment of said disease presenting with a reduced level of Hsp70 to the patient, such as Hsp70 therapies including bioactive agents that increase the intracellular concentration and/or activity of heat shock proteins, including Hsp70.

In one embodiment said method for selecting a patient further comprises a step e) administering a therapy for treatment of said disease presenting with a reduced level of Hsp70 to the individual.

In one embodiment said administering a therapy for treatment of said disease presenting with a reduced level of Hsp70 to the individual comprises administering a bioactive agent that increase the intracellular concentration and/or activity of heat shock proteins, including Hsp70, such as a Hsp70 inducer or Hsp70 protein as specified herein elsewhere.

### Monitoring disease progression

The methods as disclosed herein are also useful for monitoring disease progression of a disease presenting with a reduced level of Hsp70.

It is thus an aspect to provide a method for monitoring disease progression in an individual having a disease presenting with a reduced level of Hsp70, said method comprising the steps of
a) providing one or more PBMC samples from said individual at two or more subsequent points in time,
b) detecting Hsp70 in each of said PBMC samples,
c) quantifying or determining the level of Hsp70 in each of said PBMC samples.

In one embodiment detecting Hsp70 comprises detecting HspA1A and/or HspA1B.

In one embodiment the level of Hsp70 present in each of the PBMC samples is indicative of a progression of the disease or a remission of the disease.

It is understood that the steps of a) providing one or more PBMC samples from an individual, b) detecting Hsp70 in said PBMC samples, and c) quantifying or determining the level of Hsp70 in said PBMC samples, may each be performed according the present disclosure and specified herein elsewhere.

In one embodiment said method for monitoring disease progression comprises the further step of d) determining whether the disease presenting with a reduced level of Hsp70 is in progression or in remission.

In one embodiment said step d) comprise determining the level of Hsp70 present in the PBMC sample at an earlier time, such as t=0, and determining the level of Hsp70 present in the PBMC sample at a later time, such as t>0.

In one embodiment a first sample is taken at t=0 (earlier time) and one or more subsequent samples are taken at one or more later time points (later samples) at t>0. In one embodiment the PBMC samples are taken continuously at a certain interval.

In one embodiment the one or more subsequent samples comprise PBMC samples taken at t=0 and at 1, 2, 3, 4, 5, 6, 7, 8, 9, 10 or more time points after t=0.

In one embodiment the one or more subsequent samples are taken at one or more of t=1 day, t=2 days, t=3 days, t=4 days, t=5 days, t=6 days, t=7 days, t=14 days, t=3 weeks, t=4 weeks, t=5 weeks, t=6 weeks, t=7 weeks, t=8 weeks, t=1 month, t=2 months, t=3 months, t=4 months, t=5 months, t=6 months, t=7 months, t=8 months, t=9 months, t=10 months, t=11 months, t=12 months, t=13 months, t=14 months, t=15 months, t=16 months, t=17 months, t=18 months, t=20 months, t=22 months and/or t=24 months.

In one embodiment the one or more subsequent samples are taken at an interval of 1 day, 2 days, 3 days, 4 days, 5 days, 6 days, 7 days, 14 days, 3 weeks, 4 weeks, 5 weeks, 6 weeks, 7 weeks, 8 weeks, 1 month, 2 months, 3 months, 4 months, 5 months, 6 months, 7 months, 8 months, 9 months, 10 months, 11 months and/or 12 months. An interval of 1 month means that a subsequent sample is taken every 1 month,
In one embodiment a decrease in the level of Hsp70 over time is indicative of a progression of the disease.

In one embodiment an increase in the level of Hsp70 over time is indicative of a remission of the disease.

In one embodiment said method comprises determining whether the level of Hsp70 is lower in the subsequent sample(s), which is indicative of a progression of the disease; and/or determining whether the level of Hsp70 is higher in the subsequent sample(s), which is indicative of a remission of the disease.

In one embodiment a decrease in the level of Hsp70 over time measured at t=0 and at one or more time points at t>0 of 1 to 1000 times is indicative of a progression of the disease; such as a decrease of 1 to 2 times, 2 to 3 times, 3 to 4 times, 4 to 5 times, 5 to 6 times, 6 to 7 times, 7 to 8 times, 8 to 9 times, 9 to 10 times, 10 to 11 times, 11 to 12 times, 12 to 13 times, 13 to 14 times, 14 to 15 times, 15 to 16 times, 16 to 17 times, 17 to 18 times, 18 to 19 times, 19 to 20 times, 20 to 25 times, 25 to 30 times, 30 to 35 times, 35 to 40 times, 40 to 45 times, 45 to 50 times, 50 to 60 times, 60 to 70 times, 70 to 80 times, 80 to 90 times, 90 to 100 times, 100 to 150 times, 150 to 200 times, 200 to 250 times, 250 to 300 times, 300 to 400 times, 400 to 500 times, 500 to 600 times, 600 to 700 times, 700 to 800 times, such as a decrease in the level of Hsp70 of 900 to 1000 times.

In one embodiment an increase in the level of Hsp70 over time measured at t=0 and at one or more time points at t>0 of 1 to 1000 times is indicative of a remission of the disease; such as an increase of 1 to 2 times, 2 to 3 times, 3 to 4 times, 4 to 5 times, 5 to 6 times, 6 to 7 times, 7 to 8 times, 8 to 9 times, 9 to 10 times, 10 to 11 times, 11 to 12 times, 12 to 13 times, 13 to 14 times, 14 to 15 times, 15 to 16 times, 16 to 17 times, 17 to 18 times, 18 to 19 times, 19 to 20 times, 20 to 25 times, 25 to 30 times, 30 to 35 times, 35 to 40 times, 40 to 45 times, 45 to 50 times, 50 to 60 times, 60 to 70 times, 70 to 80 times, 80 to 90 times, 90 to 100 times, 100 to 150 times, 150 to 200 times, 200 to 250 times, 250 to 300 times, 300 to 400 times, 400 to 500 times, 500 to 600 times, 600 to 700 times, 700 to 800 times, such as an increase in the level of Hsp70 of 900 to 1000 times.

In one embodiment a decrease in the level of Hsp70 over time measured at t=0 and at t=6 months (approx.) of 500 to 20000 pg/mL PBMC is indicative of a progression of the disease, such as 500 to 750 pg/mL, such as 750 to 1000 pg/mL, such as 1000 to 1500 pg/mL, such as 1500 to 2000 pg/mL, such as 2000 to 3000 pg/mL, such as 3000 to 4000 pg/mL, such as 4000 to 5000 pg/mL, such as 5000 to 7500 pg/mL, such as 7500 to 10000 pg/mL, such as 10000 to 12500 pg/mL, such as 12500 to 15000 pg/mL, such as 15000 to 20000 pg/mL PBMC.

In one embodiment said method for monitoring disease progression in an individual having a disease presenting with a reduced level of Hsp70 comprises monitoring disease progression in an individual having a disease selected from the group consisting of a lysosomal storage disease, a neurodegenerative disease, a neuromuscular disorder, muscular dystrophy or an inflammatory muscle disorder as specified herein elsewhere.

In one embodiment said lysosomal storage disease is selected from the group consisting of lipid storage disorders including the sphingolipidoses; mucopolysaccharidoses; glycogen storage disorders; disorders of glycoprotein metabolism (glycoproteinosis); and mucolipidoses, and any subtype thereof as specified herein elsewhere. In one embodiment said lysosomal storage disease is Niemann Pick disease, such as NPC.

### Monitoring efficacy of a therapy

The methods as disclosed herein are also useful for monitoring efficacy of a treatment or a potential treatment of a disease presenting with a reduced level of Hsp70.

It is thus an aspect of the present disclosure to provide a method for monitoring efficacy of a therapy for treatment of a disease presenting with a reduced level of Hsp70 in an individual having a disease presenting with a reduced level of Hsp70, said method comprising the steps of
a) providing one or more PBMC samples from said individual before, during and/or after a therapy has been applied, maintained, reduced or elevated,
b) detecting Hsp70 in each of said one or more PBMC samples,
c) quantifying or determining the level of Hsp70 in each of said one or more PBMC samples.

In one embodiment detecting Hsp70 comprises detecting HspA1A and/or HspA1B.

In one embodiment the level of Hsp70 present in each of the PBMC samples is indicative of efficacy of a therapy for a disease presenting with a reduced level of Hsp70.

It is understood that the steps of a) providing one or more PBMC samples from an individual, b) detecting Hsp70 in said PBMC samples, and c) quantifying or determining the level of Hsp70 in said PBMC samples, may each be performed according the present disclosure and specified herein elsewhere.

In one embodiment said method for monitoring efficacy of a therapy for treatment of a disease presenting with a reduced level of Hsp70 comprises the further step of d) monitoring efficacy of a therapy for a disease presenting with a reduced level of Hsp70.

In one embodiment said step d) comprise determining the level of Hsp70 present in a PBMC sample before a therapy has been applied, maintained, reduced or elevated.

In one embodiment said step d) comprise determining the level Hsp70 present in a PBMC sample during a therapy.

In one embodiment said step d) comprise determining the level of Hsp70 present in a PBMC sample after a therapy has been applied, maintained, reduced or elevated.

In one embodiment said step d) comprise one or more of i) determining the level of Hsp70 present in a PBMC sample before a therapy has been applied, maintained, reduced or elevated; ii) determining the level of Hsp70 present in the PBMC sample during a therapy, and iii) determining the level of Hsp70 present in the PBMC sample after a therapy has been applied, maintained, reduced or elevated.

In one embodiment an increase in the level of Hsp70 after a therapy has been applied, maintained, reduced or elevated, is indicative of the therapy being efficacious.

In one embodiment a decrease in the level of Hsp70 after a therapy has been applied, maintained, reduced or elevated, is indicative of the therapy being inefficacious.

In one embodiment said method comprises one or more steps of determining whether the level of Hsp70 is higher after a therapy has been applied, maintained, reduced or elevated, which is indicative of the therapy being efficacious; and/or
one or more steps of determining whether the level of Hsp70 is lower after a therapy has been applied, maintained, reduced or elevated, which is indicative of the therapy being inefficacious.

In one embodiment an increase in the level of Hsp70 after a therapy has been applied, maintained, reduced or elevated, of 1 to 1000 times is indicative of the therapy being efficacious; such as an increase of 1 to 2 times, 2 to 3 times, 3 to 4 times, 4 to 5 times, 5 to 6 times, 6 to 7 times, 7 to 8 times, 8 to 9 times, 9 to 10 times, 10 to 11 times, 11 to 12 times, 12 to 13 times, 13 to 14 times, 14 to 15 times, 15 to 16 times, 16 to 17 times, 17 to 18 times, 18 to 19 times, 19 to 20 times, 20 to 25 times, 25 to 30 times, 30 to 35 times, 35 to 40 times, 40 to 45 times, 45 to 50 times, 50 to 60 times, 60 to 70 times, 70 to 80 times, 80 to 90 times, 90 to 100 times, 100 to 150 times, 150 to 200 times, 200 to 250 times, 250 to 300 times, 300 to 400 times, 400 to 500 times, 500 to 600 times, 600 to 700 times, 700 to 800 times, such as 900 to 1000 times

In one embodiment a decrease in the level of Hsp70 after a therapy has been applied, maintained, reduced or elevated, of 1 to 1000 times is indicative of the therapy being inefficacious; such as a decrease of 1 to 2 times, 2 to 3 times, 3 to 4 times, 4 to 5 times, 5 to 6 times, 6 to 7 times, 7 to 8 times, 8 to 9 times, 9 to 10 times, 10 to 11 times, 11 to 12 times, 12 to 13 times, 13 to 14 times, 14 to 15 times, 15 to 16 times, 16 to 17 times, 17 to 18 times, 18 to 19 times, 19 to 20 times, 20 to 25 times, 25 to 30 times, 30 to 35 times, 35 to 40 times, 40 to 45 times, 45 to 50 times, 50 to 60 times, 60 to 70 times, 70 to 80 times, 80 to 90 times, 90 to 100 times, 100 to 150 times, 150 to 200 times, 200 to 250 times, 250 to 300 times, 300 to 400 times, 400 to 500 times, 500 to 600 times, 600 to 700 times, 700 to 800 times, such as 900 to 1000 times.

In one embodiment said method for monitoring efficacy of a therapy for treatment of a disease presenting with a reduced level of Hsp70 in an individual having a disease presenting with a reduced level of Hsp70, comprises monitoring efficacy of a therapy for treatment in an individual having a disease selected from the group consisting of a lysosomal storage disease, a neurodegenerative disease, a neuromuscular disorder, muscular dystrophy or an inflammatory muscle disorder as specified herein elsewhere.

In one embodiment said lysosomal storage disease is selected from the group consisting of lipid storage disorders including the sphingolipidoses; mucopolysaccharidoses; glycogen storage disorders; disorders of glycoprotein metabolism (glycoproteinosis); and mucolipidoses, and any subtype thereof as specified herein elsewhere. In one embodiment said lysosomal storage disease is Niemann Pick disease, such as NPC.

A therapy for treatment of a disease presenting with a reduced level of Hsp70 and a bioactive agent for same purpose are disclosed herein elsewhere and included in the above methods of monitoring efficacy of a therapy.

### Sample

The methods disclosed herein comprise the provision of a peripheral blood mononuclear cell (PBMC) sample from an individual.

It is understood that the samples of the present disclosure are obtained from or obtainable from an individual, preferably a human being. In one embodiment said PBMC sample is obtained from or obtainable from an individual.

A peripheral blood mononuclear cell (PBMC) is any peripheral blood cell having a round nucleus; these cells consist of lymphocytes (T cells, B cells, NK cells), monocytes and dendritic cells. In contrast erythrocytes and platelets have no nuclei, and granulocytes (neutrophils, basophils, and eosinophils) have multi-lobed nuclei.

Thus, in one embodiment a PBMC sample is a sample comprising peripheral blood mononuclear cells having a round nucleus. In one embodiment a PBMC sample is a sample comprising lymphocytes (T cells, B cells, NK cells), monocytes and/or dendritic cells.

A PBMC sample from an individual as defined herein is in one embodiment a sample that predominantly contains PBMCs. In one embodiment a PBMC sample is a sample comprising PBMCs extracted from whole blood. In one embodiment a PBMC sample is a sample comprising or consisting essentially of the PBMC component of whole blood.

PBMCs can be extracted from whole blood using ficoll, a hydrophilic polysaccharide that separates layers of blood, and gradient centrifugation, which will separate the blood into a top layer of plasma, followed by a layer of PBMCs and a bottom fraction of polymorphonuclear cells (such as neutrophils and eosinophils) and erythrocytes. The polymorphonuclear cells can be further isolated by lysing the red blood cells. Basophils are sometimes found in both the denser and the PBMC fractions.

When peripheral whole blood is drawn for human immune system studies, it is often processed to remove red blood cells by density gradient centrifugation. Most commonly this method uses Ficoll Paque, a solution of high molecular weight sucrose polymers. Ficoll separates whole blood into two fractions above and below the density of 1.077g/ml.

Peripheral blood mononuclear cells (PBMC) are the populations of immune cells that remain at the less dense, upper interface of the Ficoll layer, often referred to as the *buffy coat* and are the cells collected when the Ficoll fractionation method is used. Erythrocytes (red blood cells) and polymorphonuclear cells (PMNs) which include neutrophils and eosinophils are generally removed during this fractionation as they are denser than 1.077g/ml. Basophils, however can be greater or less dense then 1.077g/ml and thus may be present to a small degree in the less dense PBMC fraction.

The typical composition of PBMCs include lymphocytes (T cells, B cells, and NK cells) in the range of 70 - 90% of PBMCs, monocytes in the range of 10 - 30% of PBMCs, while dendritic cells are rare, being only 1 - 2% of PBMCs. The frequencies of cell types within the lymphocyte population in some embodiments may include 70 - 85% CD3+ T cells (45 - 70% of PBMC), 5 - 20% B cells (up to 15% of PBMC), and 5 - 20% NK cells (up to 15% of PBMC).

The CD3+ compartment is composed of CD4 (25 - 60% of PBMC) and CD8 T cells (5 - 30% of PBMC), in a roughly 2:1 ratio. Both CD4 and CD8 T cells can be further subset into the naïve, and the antigen-experienced central memory, effector memory, and effector subtypes that exist in resting or activated states. Multiple markers can be used to identify these compartments to varying similarities and thus the frequencies reported using different markers may vary.

CD4 T cells are known as helper T cells and can be further classified into various functional subtypes based on the expression profiles of specific cytokines, surface markers, or transcription factors. These include regulatory T cells, TH1, TH2, and TH17 cells as well as other described subpopulations such as TH9, follicular helper, and TR1 types. The cytotoxic CD8 T cell compartment has been to shown to be extremely heterogeneous in marker expression and function and may be comprised of roughly 200 functional phenotypes.

Circulating B cells include transitional, naïve, and memory subtypes as well as plasmablasts, all of which can be found at varying populations in peripheral blood. Circulating dendritic cells include plasmacytoid dendritic cells as well as myeloid derived dendritic cells. Circulating monocytes have been described as either being classical monocytes or nonclassical CD16+ proinflammatory monocytes, which comprise up to 10% of the monocytes in peripheral blood and have unique functions compared with classical monocytes.

In one embodiment the step a) of the methods disclosed herein of providing one or more PBMC samples from an individual comprise one or more steps of:
i) providing a whole blood sample, and
ii) separating whole blood into its subcomponents to obtain a PBMC sample.

In one embodiment said whole blood sample is subject to centrifugation and/or ficoll separation to obtain a PBMC sample.

### Sample from individual

The PBMC samples provided herein in one embodiment originates from an individual having, suspected of having, at risk of having or likely to have a disease presenting with a reduced level of Hsp70.

In one embodiment said individual having, at risk of having, suspected of having or likely to have a disease presenting with a reduced level of Hsp70 is an individual having, at risk of having, suspected of having or likely to have a disease selected from the group consisting of a lysosomal storage disease, a neurodegenerative disease, a neuromuscular disorder, muscular dystrophy or an inflammatory muscle disorder as specified herein elsewhere. In one embodiment said lysosomal storage disease is selected from the group consisting of lipid storage disorders including the sphingolipidoses; mucopolysaccharidoses; glycogen storage disorders; disorders of glycoprotein metabolism (glycoproteinosis); and mucolipidoses, and any subtype thereof as specified herein elsewhere. In one embodiment said lysosomal storage disease is Niemann Pick disease, such as NPC.

In one embodiment the PBMC sample is obtained from or obtainable from an individual having, suspected of having, at risk of having, or likely to have a disease presenting with a reduced level of Hsp70.

In one embodiment the sample is obtained from an individual having, suspected of having, at risk of having, or likely to have a disease presenting with a reduced level of Hsp70, as compared to the general population. An increased risk of having or developing a disease presenting with a reduced level of Hsp70 may be based on an assessment of family history, an assessment of symptoms and/or the result of other diagnostic tests for a disease presenting with a reduced level of Hsp70.

In one embodiment the sample is obtained from an individual having one or more family members diagnosed with a disease presenting with a reduced level of Hsp70.

In one embodiment the sample is obtained from an individual having a sibling, a parent, a cousin, an uncle and/or an aunt with a disease presenting with a reduced level of Hsp70.

In one embodiment the sample is obtained from an individual having a sibling with a disease presenting with a reduced level of Hsp70.

In one embodiment the sample is obtained from an individual predisposed for developing a disease presenting with a reduced level of Hsp70.

In one embodiment the sample is obtained from an individual having one or more family members with a genetic predisposition for a disease presenting with a reduced level of Hsp70.

As specified herein elsewhere 'a disease presenting with a reduced level of Hsp70' in one embodiment includes a lysosomal storage disease, a neurodegenerative disease, a neuromuscular disorder, muscular dystrophy and an inflammatory muscle disorder.

In one embodiment the sample is obtained from an individual having one or more family members with a mutation in the *NPC1* gene and/or the *NPC2* gene.

In one embodiment the sample is obtained from an individual with one or more symptoms associated with a disease presenting with a reduced level of Hsp70; such as one or more symptoms indicative of a disease presenting with a reduced level of Hsp70.

In one embodiment the sample is obtained from an individual with one or more symptoms associated with a LSD; such as one or more symptoms indicative of a LSD.

Although the signs and symptoms vary from disease to disease, symptoms of LSD occur in each case because of a protein deficiency that inhibits the ability of the lysosomes present in each of the body's cells to perform their normal function.

In one embodiment the sample is obtained from an individual with one or more symptoms associated with Niemann Pick disease, such as Niemann Pick disease Type C; in one embodiment the symptoms are selected from the group consisting of vertical gaze palsy, enlarged liver, enlarged spleen and/or jaundice, progressive loss of motor skills, feeding difficulties, progressive learning disabilities, and seizures.

Other symptoms of LSD and NPC include deterioration both intellect and neurological functions, progressive vision failure (optic atrophy), neurological disturbances, mental deterioration, enlarged liver and/or spleen (hepatosplenomegaly), physical deterioration, progressive muscle weakness, diminished muscle tone (hypotonia), motor delays, feeding problems, respiratory difficulties and general weakness (lethargy).

### Diseases presenting with a reduced level of Hsp70

Hsp70 serves several important roles in cell homeostasis and a number of diseases present with a reduced level of Hsp70, including lysosomal storage diseases, neurodegenerative diseases, and some neuromuscular and muscular diseases.

In one embodiment, a disease presenting with a reduced level of Hsp70 as referred to herein throughout is selected from the group consisting of a lysosomal storage disease, a neurodegenerative disease, a neuromuscular disorder, muscular dystrophy and an inflammatory muscle disorder.

### Lysosomal Storage Disease

One aspect of the present disclosure relates to methods of detecting Hsp70 in PBMC samples obtained from or obtainable from an individual having, suspected of having, at risk of having, or likely to have a lysosomal storage disease; methods of diagnosing a lysosomal storage disease in an individual; methods for monitoring disease progression in an individual having a lysosomal storage disease; methods for monitoring efficacy of a therapy for treatment of a lysosomal storage disease in an individual having a lysosomal storage disease; and methods for selecting an individual having a disease presenting with a reduced level of Hsp70.

Reference to a lysosomal storage disease in the present context is meant to encompass each and any lysosomal storage disease known to the skilled person.

In one embodiment a lysosomal storage disease as disclosed herein is selected from the group consisting of lipid storage disorders (or lipidosis) including the sphingolipidoses; mucopolysaccharidoses; glycogen storage disorders; disorders of glycoprotein metabolism (glycoproteinosis); and mucolipidoses.

In one embodiment of the present disclosure there is provided methods of diagnosing a lysosomal storage disease in an individual, as outlined in detail herein above, wherein said LSD is selected from the group consisting of lipid storage disorders (or lipidosis) including the sphingolipidoses; mucopolysaccharidoses; glycogen storage disorders; disorders of glycoprotein metabolism (glycoproteinosis); and mucolipidoses; and any subtype of said LSDs specified herein.

In one embodiment of the present disclosure there is provided methods for monitoring disease progression in an individual having a lysosomal storage disease, as outlined in detail herein above, wherein said LSD is selected from the group consisting of lipid storage disorders (or lipidosis) including the sphingolipidoses; mucopolysaccharidoses; glycogen storage disorders; disorders of glycoprotein metabolism (glycoproteinosis); and mucolipidoses; and any subtype of said LSDs specified herein.

In one embodiment of the present disclosure there is provided methods for monitoring efficacy of a therapy for treatment of a lysosomal storage disease in an individual having a lysosomal storage disease, as outlined in detail herein above, wherein said LSD is selected from the group consisting of lipid storage disorders (or lipidosis) including the sphingolipidoses; mucopolysaccharidoses; glycogen storage disorders; disorders of glycoprotein metabolism (glycoproteinosis); and mucolipidoses; and any subtype of said LSDs specified herein.

In one embodiment of the present disclosure there is provided methods for selecting an individual having a lysosomal storage disease, as outlined in detail herein above, wherein said LSD is selected from the group consisting of lipid storage disorders (or lipidosis) including the sphingolipidoses; mucopolysaccharidoses; glycogen storage disorders; disorders of glycoprotein metabolism (glycoproteinosis); and mucolipidoses; and any subtype of said LSDs specified herein.

Sphingolipidoses are a heterogeneous group of inherited disorders of sphingolipid metabolism affecting primarily the central nervous system. These disorders occur chiefly in the pediatric population, and the degenerative nature of the disease processes is generally characterized by diffuse and progressive involvement of neurons (gray matter) with psychomotor retardation and myoclonus or of fiber tracts (white matter) with weakness and spasticity. The accumulated sphingolipid include gangliosides (the gangliosidoses), glycolipids/ceramide (Fabry disease, Krabbe disease), glucocerebrosides (Gaucher disease), sphingomyelin (Niemann Pick disease) and sulfatide (leukodystrohies; MLD).

In one embodiment a lysosomal storage disease as disclosed herein is a sphingolipidosis. In one embodiment a lysosomal storage disease as disclosed herein is a sphingolipidosis selected from gangliosidoses and leukodystrophies.

In one embodiment a lysosomal storage disease as disclosed herein is a gangliosidosis selected from the group consisting of Sandhoff disease (or GM2 gangliosidosis type II), classic infantile Sandhoff disease, juvenile Sandhoff disease, adult/late onset Sandhoff disease, Tay-Sachs disease (or GM2 gangliosidosis type I), infantile Tay-Sachs disease, juvenile Tay-Sachs disease, adult/late onset Tay-Sachs disease, GM2-gangliosidosis AB variant, GM1 gangliosidosis, early infantile GM1 gangliosidosis, late infantile GM1 gangliosidosis, adult GM1 gangliosidosis, GM3 gangliosidosis, and Mucolipidosis IV.

In one embodiment a lysosomal storage disease as disclosed herein is selected from the group consisting of Niemann Pick disease, Farber disease, Krabbe disease, Fabry disease, Gaucher disease, Sialidosis (Mucolipidosis type I), sulfatidosis including Metachromatic leukodystrophy (late infantile, juvenile, and adult forms) and saposin-deficiency and Multiple sulfatase deficiency (Austin disease).

In one embodiment a lysosomal storage disease as disclosed herein is Gaucher disease, including Gaucher disease type I (nonneuropathic type), type II (acute infantile neuropathic Gaucher's disease) and type III (chronic neuropathic form).

In one embodiment a lysosomal storage disease as disclosed herein is Niemann Pick disease, including Niemann Pick disease type A, Niemann Pick disease type B and Niemann Pick disease Type C.

In one embodiment a lysosomal storage disease as disclosed herein is Niemann Pick disease Type C.

In one embodiment a lysosomal storage disease as disclosed herein is a lipidosis selected from the group consisting of cerebrotendinous cholesterosis, Wolman's disease (Lysosomal acid lipase deficiency), cholesteryl ester storage disease, and neuronal ceroid lipofuscinosis (NCL). In one embodiment said neuronal ceroid lipofuscinosis is selected from the group consisting of Batten disease (Spielmeyer-Vogt disease), Bielschowsky-Jansky disease, Kufs disease, and Santavuori-Haltia disease.

In one embodiment a lysosomal storage disease as disclosed herein is a mucopolysaccharidosis selected from the group consisting of a type I mucopolysaccharidosis, a type II mucopolysaccharidosis, a type III mucopolysaccharidosis, a type IV mucopolysaccharidosis, a type VI mucopolysaccharidosis, a type VII mucopolysaccharidosis, a type VIII mucopolysaccharidosis, and a type IX mucopolysaccharidosis.

In one embodiment a lysosomal storage disease as disclosed herein is a mucopolysaccharidosis selected from the group consisting of Hurler syndrome, Hurler-Scheie syndrome and Scheie syndrome (type I); Hunter's syndrome (type II); Sanfilippo syndrome types A, B, C, or D (type III); Morquio syndrome, classic or Morquio-like (type IV); Maroteaux-Lamy syndrome, mild or severe (type VI); DiFerrante syndrome or Sly syndrome (type VII); and hyaluronidase deficiency (type IX).

In one embodiment a lysosomal storage disease as disclosed herein is a mucolipidosis selected from the group consisting of mucolipidosis type II (I-cell disease), mucolipidosis type III (pseudo-Hurler polydystrophy) and mucolipidosis type IV (mucolipidin 1 deficiency).

In one embodiment a lysosomal storage disease as disclosed herein is a glycogen storage disease selected from the group consisting of cardiac glycogenosis, Andersen disease, Cori disease (Forbes disease), Hers disease, McArdle disease, Pompe disease, Tauri disease (Tarui disease), von Gierke disease, type II Pompe disease and type IIb Danon disease.

In one embodiment a lysosomal storage disease as disclosed herein is a disorder of glycoprotein metabolism selected from the group consisting of aspartylglucosaminuria, fucosidosis, mannosidosis, alpha-mannosidosis, alpha-mannosidosis type I, alpha-mannosidosis type II, beta-mannosidosis, sialidosis type II (mucolipidosis I) and galactosialidosis.

### Neurodegenerative diseases

One aspect of the present disclosure relates to methods of detecting Hsp70 in PBMC samples obtained from or obtainable from an individual having, suspected of having, at risk of having, or likely to have a neurodegenerative disease.

Neurodegenerative diseases are a growing cause of disability in the aging community. Neurodegeneration, the slow progression of dysfunction associated with a loss of neurons and axonal connections in the central nervous system (CNS), is the primary pathological characteristic of such neurological disorders as Alzheimer's disease, Parkinson's disease (PD) and Huntington's disease (HD). This loss results in gross atrophy of the affected regions, including degeneration in the temporal lobe and parietal lobe, and parts of the frontal cortex and cingulate gyrus.

Many neurodegenerative diseases are caused by genetic mutations, most of which are located in completely unrelated genes. In many of the different diseases, the mutated gene has a common feature: a repeat of the CAG nucleotide triplet (encodes glutamine). A repeat of CAG results in a polyglutamine (polyQ) tract, and diseases showing this are known as polyglutamine diseases (polyQ diseases). These include Huntington's disease, spinocerebellar ataxias, DRPLA (Dentatorubropallidoluysian atrophy) and SBMA (Spinobulbar muscular atrophy or Kennedy disease).

In one embodiment a neurodegenerative disorder as disclosed herein is selected from the group consisting of Alzheimer's disease, Parkinson's disease, Huntington's disease, Amyotrophic lateral sclerosis (ALS; Lou Gehrig's Disease), Multiple Sclerosis, and the polyglutamine diseases including spinocerebellar ataxias (Spinocerebellar ataxia type 1, Spinocerebellar ataxia type 2, Spinocerebellar ataxia type 3 (aka Machado-Joseph's disease), Spinocerebellar ataxia type 6, Spinocerebellar ataxia type 7 and Spinocerebellar ataxia type 17), DRPLA (Dentatorubropallidoluysian atrophy) and SBMA (Spinobulbar muscular atrophy or Kennedy disease).

### Muscular diseases

One aspect of the present disclosure relates to methods of detecting Hsp70 in PBMC samples obtained from or obtainable from an individual having, suspected of having, at risk of having, or likely to have a muscular disease, such as a muscular disease selected from the group consisting of a neuromuscular disorder, muscular dystrophy and an inflammatory muscle disorder.

### Neuromuscular disorders

Neuromuscular disorders affect the nerves that control your voluntary muscles. Voluntary muscles are the ones you can control, like in your arms and legs. Your nerve cells, also called neurons, send the messages that control these muscles. When the neurons become unhealthy or die, communication between your nervous system and muscles breaks down. As a result, your muscles weaken and waste away. The weakness can lead to twitching, cramps, aches and pains, and joint and movement problems. Sometimes it also affects heart function and your ability to breathe. Some examples of central neuromuscular disorders include cerebrovascular accident, Parkinson's disease, multiple sclerosis, Huntington's disease and Creutzfeldt-Jakob disease. Spinal muscular atrophies are disorders of lower motor neuron while amyotrophic lateral sclerosis is a mixed upper and lower motor neuron condition.

In one embodiment the neuromuscular disorder is a central neuromuscular disorder.

In one embodiment the neuromuscular disorder is selected from the group consisting of Amyotrophic lateral sclerosis (ALS), Multiple Sclerosis, Parkinson's disease, Huntington's disease, Creutzfeldt-Jakob disease, Myasthenia gravis, Spinal Muscular Atrophy (SMA), Spinal muscular atrophy with respiratory distress type 1 (SMARD1; aka. Distal spinal muscular atrophy type 1 (DSMA1)), Congenital myasthenic syndrome (CMS), Congenital myopathy, Cramp fasciculation syndrome, Muscular dystrophies, Hereditary spastic paraplegia, Inclusion body myositis, Neuromyotonia (NMT, aka Isaacs syndrome, Isaacs-Merton syndrome), Mitochondrial myopathy, Lambert-Eaton myasthenic syndrome (LEMS), Myotonic dystrophy, Peripheral neuropathy, Spinal and bulbar muscular atrophy (SBMA, or Kennedy's disease), Stiff person syndrome and Guillain-Barré syndrome.

In one embodiment the Spinal Muscular Atrophy (SMA) is selected from the group consisting of SMA1 (infantile, Werdnig-Hoffmann disease), SMA type 0 (or, severe infantile SMA), SMA2 (intermediate, Dubowitz disease), SMA3 (juvenile, Kugelberg-Welander disease) and SMA4 (adult-onset).

In one embodiment the Congenital myasthenic syndrome (CMS) is selected from the group consisting of presynaptic CMS, synaptic CMS and postsynaptic CMS.

In one embodiment the Congenital myopathy is selected from the group consisting of Nemaline myopathy, Myotubular myopathy, Central core disease or central core myopathy, Congenital fiber type disproportion and Cylindrical spirals myopathy.

In one embodiment the Pompe disease is selected from the group consisting of infantile form and late onset form.

In one embodiment the Hereditary spastic paraplegia is selected from the group consisting of MASA syndrome, Pelizaeus-Merzbacher disease, Strumpell disease, Cataracts with motor neuronopathy, short stature and skeletal abnormalities, Troyer syndrome, MAST syndrome, Allan-Herndon-Dudley syndrome, Lison syndrome, Spastic ataxia 2, SPOAN syndrome, Martsolf syndrome or Warburg Micro syndrome, Kufor-Rakeb syndrome, MEGDEL syndrome and Harel-Yoon syndrome.

In one embodiment the Inclusion body myositis is selected from the group consisting of sporadic Inclusion body myositis (sIBM) and hereditary Inclusion body myositis (hIBM), wherein said hIBM includes IBM2, IBM3 and Inclusion body myopathy with early-onset Paget disease and frontotemporal dementia (IBMPFD).

In one embodiment the Neuromyotonia (NMT) is selected from the group consisting of Chronic, Monophasic and Relapsing Remitting.

In one embodiment the Mitochondrial myopathy is selected from the group consisting of Kearns-Sayre syndrome (KSS), Myoclonic epilepsy and ragged-red fibers (MERRF) Mitochondrial encephalomyopathy, lactic acidosis, and stroke-like syndrome (MELAS), and Chronic progressive external ophthalmoplegia (CPEO).

In one embodiment the Myotonic dystrophy is Myotonic dystrophy type 1 (DM1) or Myotonic dystrophy type 2 (DM2).

In one embodiment the Peripheral neuropathy is selected from the group consisting of Mononeuropathy, Polyneuropathy, Mononeuritis multiplex (or polyneuritis multiplex), Autonomic neuropathy and Neuritis.

In one embodiment the Guillain-Barré syndrome is selected from the group consisting of Acute inflammatory demyelinating polyneuropathy (AIDP), Acute motor axonal neuropathy (AMAN), Acute motor and sensory axonal neuropathy (AMSAN), Pharyngeal-cervical-brachial variant and Miller Fisher syndrome.

### Muscular dystrophies

Muscular dystrophy (MD) is a group of muscle diseases that results in increasing weakening and breakdown of skeletal muscles over time. The disorders differ in which muscles are primarily affected, the degree of weakness, how fast they worsen, and when symptoms begin. Many people will eventually become unable to walk. Some types are also associated with problems in other organs. There are nine main categories of muscular dystrophy that contain more than thirty specific types.

In one embodiment the Muscular dystrophy is selected from the group consisting of Duchenne muscular dystrophy (DMD), Becker muscular dystrophy, Congenital muscular dystrophy, Distal muscular dystrophy, Emery-Dreifuss muscular dystrophy Facioscapulohumeral muscular dystrophy, Limb-girdle muscular dystrophy, Myotonic muscular dystrophy and Oculopharyngeal muscular dystrophy.

### Inflammatory muscle disorder

In one embodiment the inflammatory muscle disorder is selected from the group consisting of Inflammatory myopathy (inflammatory muscle disease or myositis), idiopathic Inflammatory myopathy, Polymyositis (PM) , dermatomyositis (DM), Inclusion-body myositis (sIBM and hIBM), Polymyalgia rheumatica (or "muscle rheumatism") and Rhabdomyolysis.

### Therapies for treatment

According to the present disclosure there is provided methods of administering a therapy for treatment of a disease presenting with a reduced level of Hsp70 to a patient diagnosed with a disease presenting with a reduced level of Hsp70.

In one embodiment step e) of administering a therapy for treatment of a disease presenting with a reduced level of Hsp70 to a patient diagnosed with said disease presenting with a reduced level of Hsp70 comprises administering an effective amount of a bioactive agent to said individual.

A therapy for treatment of a disease presenting with a reduced level of Hsp70 and a bioactive agent for same purpose includes known therapies for disease presenting with a reduced level of Hsp70.

A "Bioactive agent" (i. e., biologically active substance/agent) is any agent, drug, substance, compound, composition of matter or mixture which provides some pharmacologic, often beneficial, effect that can be demonstrated *in vivo* or *in vitro*. As used herein, this term further includes any physiologically or pharmacologically active substance that produces a localized or systemic effect in an individual.

### Hsp70 and Hsp inducers

In one embodiment step e) of administering a therapy for treatment of a disease presenting with a reduced level of Hsp70 to a patient diagnosed with said disease presenting with a reduced level of Hsp70 comprises administering an effective amount of a bioactive agent that increase the intracellular concentration and/or activity of heat shock proteins, such as Hsp70.

In one embodiment a therapy for treatment of a disease presenting with a reduced level of Hsp70 is a bioactive agent that increases the intracellular concentration (or levels) and/or activity of one or more heat shock proteins, including Hsp70 and co-chaperones; and includes Hsp70 itself, or a functional fragment or variant thereof, any heat shock protein inducer and any Hsp70 inducer known to the skilled person.

A bioactive agent that increases the intracellular concentration and/or activity of one or more heat shock proteins, including Hsp70, and a bioactive agent that increases the intracellular concentration and/or activity of Hsp70, can be used interchangeably with 'Hsp70 inducer' herein.

An Hsp70 inducer can amplify Hsp70 gene expression and protein expression with or without a concomitant stress. A direct Hsp70 inducer is a compound that can by itself amplify Hsp70 gene expression and protein expression without a concomitant stress. An indirect Hsp70 inducer, or an Hsp70 co-inducer, is a compound that cannot amplify Hsp70 gene expression and protein expression without a concomitant (mild) stress, but the stress-induced increase in Hsp70 levels is further elevated or enhanced by their presence.

It follows that a bioactive agent may increase the intracellular concentration and/or activity of heat shock proteins, such as Hsp70, either directly or indirectly.

In one embodiment, the bioactive agent is Hsp70 protein, or a functional fragment or variant thereof. In one embodiment said Hsp70 protein is selected from HSPA1A and HSPA1B, or a functional fragment or variant thereof. In one embodiment said functional fragment or variant of Hsp70 has at least 75% sequence identity to Hsp70 such as to any one of HSPA1A and HSPA1B, such as at least 80%, at least 85%, at least 90%, at least 95% or at least 99% sequence identity.

In another embodiment, the bioactive agent is an inducer of heat shock proteins, including Hsp70.

In one embodiment the inducer of heat shock proteins, including Hsp70, is an inducer of one or more of Hsp70, Hsp40, Hsp72 and Hsp90, and co-chaperones.

In one embodiment the inducer of heat shock proteins is an inducer of at least Hsp70. In one embodiment the inducer of heat shock proteins is an inducer of Hsp70.

Reference to an inducer of Hsp70, or inducing Hsp70, implies that at least Hsp70 is induced, and does not exclude co-induction of other proteins and effectors such as other heat shock proteins. An inducer of Hsp70 refers equally to Hsp70 inducers and co-inducers, and direct and indirect Hsp70 inducers.

In one embodiment, the bioactive agent comprises a combination of Hsp70, or a functional fragment or variant thereof, and an inducer of heat shock proteins including Hsp70.

In one embodiment the bioactive agent activates the heat shock response. In one embodiment the bioactive agent increases the intracellular concentration and/or activity of one or more heat shock proteins, including Hsp70. In one embodiment the bioactive agent increases the intracellular concentration (or level) and/or activity of Hsp70. In one embodiment the bioactive agent increases the intracellular concentration (or level) of Hsp70. In one embodiment the bioactive agent is an inducer of one or more heat shock proteins, including Hsp70. In one embodiment the bioactive agent is an inducer of Hsp70.

### Small molecule inducers of heat shock proteins

In one embodiment the bioactive agent is a small molecule inducer of heat shock proteins, including Hsp70, such as a small molecule inducer of Hsp70.

In one embodiment a small molecule inducer of one or more heat shock proteins, including Hsp70; is a compound capable of increasing the intracellular concentration (or level) of *inter alia* Hsp70, such as by amplifying Hsp70 gene expression.

In one embodiment the bioactive agent is capable of increasing the intracellular concentration (or levels) of Hsp70 by amplifying Hsp70 gene expression. In one embodiment the bioactive agent is capable of increasing the intracellular concentration (or level) of Hsp70 by amplifying Hsp70 gene expression, wherein said bioactive agent is a hydroxylamine derivative, such as a hydroxylamine derivative small molecule.

Examples of such hydroxylamine derivatives include arimoclomol, iroxanadine, bimoclomol, BGP-15, their stereoisomers and the acid addition salts thereof.

### Arimoclomol:

In one embodiment the small molecule inducer of Hsp70 is selected from N-[2-hydroxy-3-(1-piperidinyl)-propoxy]-pyridine-1-oxide-3-carboximidoyl chloride (arimoclomol), its stereoisomers and the acid addition salts thereof. Arimoclomol is further described in e.g. WO 00/50403.

In one embodiment the small molecule inducer of Hsp70 is selected from N-[2-hydroxy-3-(1-piperidinyl)-propoxy]-pyridine-1-oxide-3-carboximidoyl chloride (arimoclomol), its optically active (+) or (-) enantiomer, a mixture of the enantiomers of any ratio, and the racemic compound, furthermore, the acid addition salts formed from any of the above compounds with mineral or organic acids constitute objects of the present disclosure. All possible geometrical isomer forms of N-[2-hydroxy-3-(1-piperidinyl)-propoxy]-pyridine-1-oxide-3-carboximidoyl chloride belong to the scope of the disclosure. The term "the stereoisomers of N-[2-hydroxy-3-(1-piperidinyl)-propoxy]-pyridine-1-oxide-3-carboximidoyl chloride" refers to all possible optical and geometrical isomers of the compound.

If desired, the N-[2-hydroxy-3-(1-piperidinyl)-propoxy]-pyridine-1-oxide-3-carboximidoyl chloride or one of its optically active enantiomers can be transformed into an acid addition salt with a mineral or organic acid, by known methods.

In one embodiment the small molecule inducer of Hsp70 is the racemate of N-[2-hydroxy-3-(1-piperidinyl)-propoxy]-pyridine-1-oxide-3-carboximidoyl chloride.

In one embodiment the small molecule inducer of Hsp70 is an optically active stereoisomer of N-[2-hydroxy-3-(1-piperidinyl)-propoxy]-pyridine-1-oxide-3-carboximidoyl chloride.

In one embodiment the small molecule inducer of Hsp70 is an enantiomer of N-[2-hydroxy-3-(1-piperidinyl)-propoxy]-pyridine-1-oxide-3-carboximidoyl chloride.

In one embodiment the small molecule inducer of Hsp70 is selected from the group consisting of (+)-R-N-[2-hydroxy-3-(1-piperidinyl)-propoxy]-pyridine-1-oxide-3-carboximidoyl chloride and (-)-(S)-N-[2-hydroxy-3-(1-piperidinyl)-propoxy]-pyridine-1-oxide-3-carboximidoyl chloride.

In one embodiment the small molecule inducer of Hsp70 is an acid addition salt of N-[2-hydroxy-3-(1-piperidinyl)-propoxy]-pyridine-1-oxide-3-carboximidoyl chloride.

In one embodiment the small molecule inducer of Hsp70 is selected from the group consisting of N-[2-hydroxy-3-(1-piperidinyl)-propoxy]-pyridine-1-oxide-3-carboximidoyl chloride citrate (BRX-345), and N-[2-hydroxy-3-(1-piperidinyl)-propoxy]-pyridine-1-oxide-3-carboximidoyl chloride maleate (BRX-220).

In one embodiment the small molecule inducer of Hsp70 is selected from the group consisting of (+)-R-N-[2-hydroxy-3-(1-piperidinyl)-propoxy]-pyridine-1-oxide-3-carboximidoyl chloride citrate; (*-)-S-N*-[2-hydroxy-3-(1-piperidinyl)-propoxy]-pyridine-1-oxide-3-carboximidoyl chloride citrate; (+)-R-N-[2-hydroxy-3-(1-piperidinyl)-propoxy]-pyridine-1-oxide-3-carboximidoyl chloride maleate; and (*-)-S-N*-[2-hydroxy-3-(1-piperidinyl)-propoxy]-pyridine-1-oxide-3-carboximidoyl chloride maleate.

### BGP-15:

In one embodiment the small molecule inducer of Hsp70 is N-[2-hydroxy-3-(1-piperidinyl)propoxy]-3-pyridinecarboximidamide, dihydrochloride (BGP-15), its stereoisomers and the acid addition salts thereof.

In one embodiment the small molecule inducer of Hsp70 is selected from N-[2-hydroxy-3-(1-piperidinyl)propoxy]-3-pyridinecarboximidamide, dihydrochloride (BGP-15), its optically active (+) or (-) enantiomer, a mixture of the enantiomers of any ratio, and the racemic compound, furthermore, the acid addition salts formed from any of the above compounds with mineral or organic acids constitute objects of the present disclosure. All possible geometrical isomer forms of N-[2-hydroxy-3-(1-piperidinyl)propoxy]-3-pyridinecarboximidamide, dihydrochloride belong to the scope of the disclosure. The term "the stereoisomers of N-[2-hydroxy-3-(1-piperidinyl)propoxy]-3-pyridine-carboximidamide, dihydrochloride" refers to all possible optical and geometrical isomers of the compound.

### Iroxanadine:

In one embodiment the small molecule inducer of Hsp70 is selected from 5,6-dihydro-5-(1-piperidinyl)methyl-3-(3-pyridyl)-4H-1,2,4-oxadiazine (iroxanadine), its stereoisomers and the acid addition salts thereof. Iroxanadine is further described in e.g. WO 97/16439 and WO 00/35914.

In one embodiment the small molecule inducer of Hsp70 is selected from 5,6-dihydro-5-(1-piperidinyl)methyl-3-(3-pyridyl)-4H-1,2,4-oxadiazine (iroxanadine), its optically active (+) or (-) enantiomer, a mixture of the enantiomers of any ratio, and the racemic compound, furthermore, the acid addition salts formed from any of the above compounds with mineral or organic acids constitute objects of the present disclosure. All possible geometrical isomer forms of 5,6-dihydro-5-(1-piperidinyl)methyl-3-(3-pyridyl)-4H-1,2,4-oxadiazine belong to the scope of the disclosure. The term "the stereoisomers of 5,6-dihydro-5-(1-piperidinyl)methyl-3-(3-pyridyl)-4H-1,2,4-oxadiazine" refers to all possible optical and geometrical isomers of the compound.

If desired, the 5,6-dihydro-5-(1-piperidinyl)methyl-3-(3-pyridyl)-4H-1,2,4-oxadiazine or one of its optically active enantiomers can be transformed into an acid addition salt with a mineral or organic acid, by known methods.

In one embodiment the small molecule inducer of Hsp70 is the racemate of 5,6-dihydro-5-(1-piperidinyl)methyl-3-(3-pyridyl)-4H-1,2,4-oxadiazine.

In one embodiment the small molecule inducer of Hsp70 is an optically active stereoisomer of 5,6-dihydro-5-(1-piperidinyl)methyl-3-(3-pyridyl)-4H-1,2,4-oxadiazine.

In one embodiment the small molecule inducer of Hsp70 is an enantiomer of 5,6-dihydro-5-(1-piperidinyl)methyl-3-(3-pyridyl)-4H-1,2,4-oxadiazine.

In one embodiment the small molecule inducer of Hsp70 is selected from the group consisting of (+)-5,6-dihydro-5-(1-piperidinyl)methyl-3-(3-pyridyl)-4H-1,2,4-oxadiazine and (-)-5,6-dihydro-5-(1-piperidinyl)methyl-3-(3-pyridyl)-4H-1,2,4-oxadiazine.

In one embodiment the small molecule inducer of Hsp70 is an acid addition salt of 5,6-dihydro-5-(1-piperidinyl)methyl-3-(3-pyridyl)-4H-1,2,4-oxadiazine.

In one embodiment the small molecule inducer of Hsp70 is selected from the group consisting of 5,6-dihydro-5-(1-piperidinyl)methyl-3-(3-pyridyl)-4H-1,2,4-oxadiazine citrate, and 5,6-dihydro-5-(1-piperidinyl)methyl-3-(3-pyridyl)-4H-1,2,4-oxadiazine maleate.

In one embodiment the small molecule inducer of Hsp70 is selected from the group consisting of (+)-5,6-dihydro-5-(1-piperidinyl)methyl-3-(3-pyridyl)-4H-1,2,4-oxadiazine citrate; (-)-5,6-dihydro-5-(1-piperidinyl)methyl-3-(3-pyridyl)-4H-1,2,4-oxadiazine citrate; (+)-5,6-dihydro-5-(1-piperidinyl)methyl-3-(3-pyridyl)-4H-1,2,4-oxadiazine maleate; and (-)-5,6-dihydro-5-(1-piperidinyl)methyl-3-(3-pyridyl)-4H-1,2,4-oxadiazine maleate.

### Bimoclomol:

In one embodiment the small molecule inducer of Hsp70 is selected from N-[2-hydroxy-3-(1-piperidinyl)-propoxy]-3-pyridinecarboximidoyl chloride (bimoclomol) its stereoisomers and the acid addition salts thereof. Bimoclomol is further described in e.g. WO 1997/16439.

In one embodiment the small molecule inducer of Hsp70 is selected from N-[2-hydroxy-3-(1-piperidinyl)-propoxy]-3-pyridinecarboximidoyl chloride (bimoclomol), its optically active (+) or (-) enantiomer, a mixture of the enantiomers of any ratio, and the racemic compound, furthermore, the acid addition salts formed from any of the above compounds with mineral or organic acids constitute objects of the present disclosure. All possible geometrical isomer forms of N-[2-hydroxy-3-(1-piperidinyl)-propoxy]-3-pyridinecarboximidoyl chloride belong to the scope of the disclosure. The term "the stereoisomers of N-[2-hydroxy-3-(1-piperidinyl)-propoxy]-3-pyridinecarboximidoyl chloride" refers to all possible optical and geometrical isomers of the compound.

If desired, the N-[2-hydroxy-3-(1-piperidinyl)-propoxy]-3-pyridinecarboximidoyl chloride or one of its optically active enantiomers can be transformed into an acid addition salt with a mineral or organic acid, by known methods.

In one embodiment the small molecule inducer of Hsp70 is the racemate of N-[2-hydroxy-3-(1-piperidinyl)-propoxy]-3-pyridinecarboximidoyl chloride.

In one embodiment the small molecule inducer of Hsp70 is an optically active stereoisomer of N-[2-hydroxy-3-(1-piperidinyl)-propoxy]-3-pyridinecarboximidoyl chloride.

In one embodiment the small molecule inducer of Hsp70 is an enantiomer of N-[2-hydroxy-3-(1-piperidinyl)-propoxy]-3-pyridinecarboximidoyl chloride.

In one embodiment the small molecule inducer of Hsp70 is selected from the group consisting of (+)-R-N-[2-hydroxy-3-(1-piperidinyl)-propoxy]-3-pyridinecarboximidoyl chloride and (-)-(S)-N-[2-hydroxy-3-(1-piperidinyl)-propoxy]-3-pyridinecarboximidoyl chloride.

In one embodiment the small molecule inducer of Hsp70 is an acid addition salt of N-[2-hydroxy-3-(1-piperidinyl)-propoxy]-3-pyridinecarboximidoyl chloride.

In one embodiment the small molecule inducer of Hsp70 is selected from the group consisting of N-[2-hydroxy-3-(1-piperidinyl)-propoxy]-3-pyridinecarboximidoyl chloride citrate, and N-[2-hydroxy-3-(1-piperidinyl)-propoxy]-3-pyridinecarboximidoyl chloride maleate.

In one embodiment the small molecule inducer of Hsp70 is selected from the group consisting of (+)-R-N-[2-hydroxy-3-(1-piperidinyl)-propoxy]-3-pyridinecarboximidoyl chloride citrate; (*-)-S-*N-[2-hydroxy-3-(1-piperidinyl)-propoxy]-3-pyridinecarboximidoyl chloride citrate; (+)-R-N-[2-hydroxy-3-(1-piperidinyl)-propoxy]-3-pyridinecarboximidoyl chloride maleate; and (*-)-S-*N-[2-hydroxy-3-(1-piperidinyl)-propoxy]-3-pyridine-carboximidoyl chloride maleate.

A number of compounds have been shown to induce (or co-induce) HSPs, including Hsp70. In one embodiment the inducer of Hsp70 is selected from the group consisting of: membrane-interactive compounds such as alkyllysophospholipid edelfosine (ET-18-OCH3 or 1-octadecyl-2-methyl-rac-glycero-3-phosphocholine); anti-inflammatory drugs including cyclooxygenase 1/2 inhibitors such as celecoxib and rofecoxib, as well as NSAIDs such as acetyl-salicylic acid, sodium salicylate and indomethacin; dexamethasone; prostaglandins PGA1, PGj2 and 2-cyclopentene-1-one; peroxidase proliferator-activated receptor-gamma agonists; tubulin-interacting anticancer agents including vincristine and paclitaxel; the insulin sensitizer pioglitazone; anti-neoplastic agents such as carboplatin, doxorubicin, fludarabine, ifosfamide and cytarabine; Hsp90 inhibitors including geldanamycin, 17-AAG, 17-DMAG, radicicol, herbimycin-A and arachidonic acid; proteasome inhibitors such as MG132, lactacystin, Bortezomib, Carfilzomib and Oprozomib; serine protease inhibitors such as DCIC, TLCK and TPCK; Histone Deacetylase Inhibitors (HDACi) including SAHA/vorinostat, Belinostat/PXD101, LB-205, LBH589 (panobinostat), FK-228, CI-994, trichostatin A (TSA) and PCI-34051; anti-ulcer drugs including geranylgeranylacetone (GGA), rebamipide, carbenoxolone and polaprezinc (zinc L-carnosine); heavy metals (zinc and tin); cocaine; nicotine; alcohol; alpha-adrenergic agonists; cyclopentenone prostanoids; L-type Ca++ channel blockers, such as L-type Ca++ channel blockers that also inhibits ryanodine receptors, such as lacidipine; ryanodine receptor antagonists such as DHBP (1,1'-diheptyl- 4,4'-bipyridium; as well as herbal medicines including paeoniflorin, glycyrrhizin, celastrol, dihydrocelastrol, dihydrocelastrol diacetate and curcumin.

In one embodiment the inducer of Hsp70 is a proteasome inhibitor. In one embodiment the proteasome inhibitor is selected from the group consisting of Bortezomib, Carfilzomib, Oprozomib, MG132 and lactacystin.

In one embodiment the inducer of Hsp70 is a HDAC inhibitor. In one embodiment the HDACi is selected form the group consisting of SAHA/vorinostat, Belinostat/PXD101, LB-205, LBH589 (panobinostat), FK-228, CI-994, trichostatin A (TSA) and PCI-34051.

In one embodiment the inducer of Hsp70 is a membrane fluidizer. Treatment with a membrane fluidizer may also be termed lipid therapy. In one embodiment the inducer of Hsp70 is a membrane fluidizer selected from the group consisting of benzyl alcohol, heptanol, AL721, docosahexaenoic acid, aliphatic alcohols, oleyl alcohol, dimethylaminoethanol, A₂C, farnesol and anaesthetics such as lidocaine, ropivacaine, bupivacaine and mepivacaine, as well as others known to the skilled person.

### Other treatments of LSD

In addition to the above described treatments with Hsp or Hsp inducing agents, LSD may be treated by other means as described herein below. Such treatments may be combined with the treatment with Hsp and/or Hsp inducers.

The underlying cause of LSDs is the inability of specific lysosomal enzymes to catabolize efficiently specific lysosomal substances such as lipids. Therefore the use of enzyme replacement therapy (ERT), by providing to a patient the recombinant enzyme, has been employed for a subset of these diseases, including Gaucher and Fabry disease. ERT is effective only towards the specific type of disease to which the recombinant enzyme has been produced.

In one embodiment step e) of administering a therapy for treatment of a lysosomal storage disease to a patient diagnosed with said lysosomal storage disease comprises administering an effective amount of an enzyme replacement therapy (ERT).

In one embodiment step e) of administering a therapy for treatment of a lysosomal storage disease to a patient diagnosed with said lysosomal storage disease comprises administering a substrate reduction therapy (SRT).

In one embodiment step e) of administering a therapy for treatment of a lysosomal storage disease to a patient diagnosed with said lysosomal storage disease comprises administering a therapy selected from the group consisting of pain relievers; corticosteroids; a transplantation, such as bone marrow transplantation, cord blood transplantation or stem cell transplantation; and symptomatic and supportive therapy, such as physical therapy.

In one embodiment the LSD in the present methods is Niemann Pick disease, and the therapy for treatment of Niemann Pick disease is selected from the group consisting of a small molecule inducer of heat shock proteins including Hsp70, such as arimoclomol; Ambroxol and Miglustat.

In one embodiment the LSD in the present methods is Gaucher disease, and the therapy for treatment of Gaucher disease is selected from the group consisting of enzyme replacement therapy, including intravenous recombinant glucosylceramidase and Cerezyme^{®} (imiglucerase for injection); Miglustat; Ambroxol; bone marrow transplantation; surgery; blood transfusion; joint replacement surgery; antibiotics; antiepileptics; bisphosphonates and liver transplant.

In another embodiment the LSD in the present methods is Fabry disease, and the therapy for treatment of Fabry disease is selected from the group consisting of enzyme replacement therapy, including Fabrazyme^{®} (agalsidase beta) and Replagal (Agalsidase alpha).

### Sequences

SEQ ID NO: 1: The protein sequence for Homo sapiens heat shock 70 kDa protein 1A (HSPA1A_HUMAN) (NM_005345.5 / UniProtKB - P0DMV8): 
MAKAAAIGIDLGTTYSCVGVFQHGKVEIIANDQGNRTTPSYVAFTDTERLIGDAAKNQVALNPQNTVFDA

KRLIGRKFGDPVVQSDMKHWPFQVINDGDKPKVQVSYKGETKAFYPEEISSMVLTKMKEIAEAYLGYPVT

NAVITVPAYFNDSQRQATKDAGVIAGLNVLRIINEPTAAAIAYGLDRTGKGERNVLIFDLGGGTFDVSIL

TIDDGIFEVKATAGDTHLGGEDFDNRLVNHFVEEFKRKHKKDISQNKRAVRRLRTACERAKRTLSSSTQA

SLEIDSLFEGIDFYTSITRARFEELCSDLFRSTLEPVEKALRDAKLDKAQIHDLVLVGGSTRIPKVQKLL

QDFFNGRDLNKSINPDEAVAYGAAVQAAILMGDKSENVQDLLLLDVAPLSLGLETAGGVMTALIKRNSTI

PTKQTQIFTTYSDNQPGVLIQVYEGERAMTKDNNLLGRFELSGIPPAPRGVPQIEVTFDIDANGILNVTA

TDKSTGKANKITITNDKGRLSKEEIERMVQEAEKYKAEDEVQRERVSAKNALESYAFNMKSAVEDEGLKG

KISEADKKKVLDKCQEVISWLDANTLAEKDEFEHKRKELEQVCNPIISGLYQGAGGPGPGGFGAQGPKGG

SGSGPTIEEVD
SEQ ID NO: 2: The initiator methionine (M at position 1) of SEQ ID NO:1 is removed to yield a 640-amino acid long sequence (position 2-641): 
AKAAAIGIDLGTTYSCVGVFQHGKVEIIANDQGNRTTPSYVAFTDTERLIGDAAKNQVALNPQNTVFDAK
RLIGRKFGDPVVQSDMKHWPFQVINDGDKPKVQVSYKGETKAFYPEEISSMVLTKMKEIAEAYLGYPVTN
AVITVPAYFNDSQRQATKDAGVIAGLNVLRIINEPTAAAIAYGLDRTGKGERNVLIFDLGGGTFDVSILT
IDDGIFEVKATAGDTHLGGEDFDNRLVNHFVEEFKRKHKKDISQNKRAVRRLRTACERAKRTLSSSTQAS
LEIDSLFEGIDFYTSITRARFEELCSDLFRSTLEPVEKALRDAKLDKAQIHDLVLVGGSTRIPKVQKLLQ
DFFNGRDLNKSINPDEAVAYGAAVQAAILMGDKSENVQDLLLLDVAPLSLGLETAGGVMTALIKRNSTIP
TKQTQIFTTYSDNQPGVLIQVYEGERAMTKDNNLLGRFELSGIPPAPRGVPQIEVTFDIDANGILNVTAT
DKSTGKANKITITNDKGRLSKEEIERMVQEAEKYKAEDEVQRERVSAKNALESYAFNMKSAVEDEGLKGK
ISEADKKKVLDKCQEVISWLDANTLAEKDEFEHKRKELEQVCNPIISGLYQGAGGPGPGGFGAQGPKGGS
GSGPTIEEVD
SEQ ID NO: 3: The nucleic acid (DNA) sequence for Homo sapiens heat shock 70 kDa protein 1A (HSPA1A) (NM_005345.5): 
1 ataaaagccc aggggcaagc ggtccggata acggctagcc tgaggagctg ctgcgacagt

61 ccactacctt tttcgagagt gactcccgtt gtcccaaggc ttcccagagc gaacctgtgc

121 ggctgcaggc accggcgcgt cgagtttccg gcgtccggaa ggaccgagct cttctcgcgg

181 atccagtgtt ccgtttccag cccccaatct cagagcggag ccgacagaga gcagggaacc

241 ggcatggcca aagccgcggc gatcggcatc gacctgggca ccacctactc ctgcgtgggg

301 gtgttccaac acggcaaggt ggagatcatc gccaacgacc agggcaaccg caccaccccc

361 agctacgtgg ccttcacgga caccgagcgg ctcatcgggg atgcggccaa gaaccaggtg

421 gcgctgaacc cgcagaacac cgtgtttgac gcgaagcggc tgattggccg caagttcggc

481 gacccggtgg tgcagtcgga catgaagcac tggcctttcc aggtgatcaa cgacggagac
 
541 aagcccaagg tgcaggtgag ctacaagggg gagaccaagg cattctaccc cgaggagatc
601 tcgtccatgg tgctgaccaa gatgaaggag atcgccgagg cgtacctggg ctacccggtg
661 accaacgcgg tgatcaccgt gccggcctac ttcaacgact cgcagcgcca ggccaccaag
721 gatgcgggtg tgatcgcggg gctcaacgtg ctgcggatca tcaacgagcc cacggccgcc
781 gccatcgcct acggcctgga cagaacgggc aagggggagc gcaacgtgct catctttgac
841 ctgggcgggg gcaccttcga cgtgtccatc ctgacgatcg acgacggcat cttcgaggtg
901 aaggccacgg ccggggacac ccacctgggt ggggaggact ttgacaacag gctggtgaac
961 cacttcgtgg aggagttcaa gagaaaacac aagaaggaca tcagccagaa caagcgagcc
1021 gtgaggcggc tgcgcaccgc ctgcgagagg gccaagagga ccctgtcgtc cagcacccag
1081 gccagcctgg agatcgactc cctgtttgag ggcatcgact tctacacgtc catcaccagg
1141 gcgaggttcg aggagctgtg ctccgacctg ttccgaagca ccctggagcc cgtggagaag
1201 gctctgcgcg acgccaagct ggacaaggcc cagattcacg acctggtcct ggtcgggggc
1261 tccacccgca tccccaaggt gcagaagctg ctgcaggact tcttcaacgg gcgcgacctg
1321 aacaagagca tcaaccccga cgaggctgtg gcctacgggg cggcggtgca ggcggccatc
1381 ctgatggggg acaagtccga gaacgtgcag gacctgctgc tgctggacgt ggctcccctg
1441 tcgctggggc tggagacggc cggaggcgtg atgactgccc tgatcaagcg caactccacc
1501 atccccacca agcagacgca gatcttcacc acctactccg acaaccaacc cggggtgctg
1561 atccaggtgt acgagggcga gagggccatg acgaaagaca acaatctgtt ggggcgcttc
1621 gagctgagcg gcatccctcc ggcccccagg ggcgtgcccc agatcgaggt gaccttcgac
1681 atcgatgcca acggcatcct gaacgtcacg gccacggaca agagcaccgg caaggccaac
1741 aagatcacca tcaccaacga caagggccgc ctgagcaagg aggagatcga gcgcatggtg
1801 caggaggcgg agaagtacaa agcggaggac gaggtgcagc gcgagagggt gtcagccaag
1861 aacgccctgg agtcctacgc cttcaacatg aagagcgccg tggaggatga ggggctcaag
1921 ggcaagatca gcgaggcgga caagaagaag gtgctggaca agtgtcaaga ggtcatctcg
1981 tggctggacg ccaacacctt ggccgagaag gacgagtttg agcacaagag gaaggagctg
2041 gagcaggtgt gtaaccccat catcagcgga ctgtaccagg gtgccggtgg tcccgggcct
2101 gggggcttcg gggctcaggg tcccaaggga gggtctgggt caggccccac cattgaggag
2161 gtagattagg ggcctttcca agattgctgt tttgttttgg gagcttcaag actttgcatt
2221 tcctagtatt tctgtttgtc agttctcaat ttcctgtgtt tgcaatgttg aaattttttg
2281 gtgaagtact gaacttgctt tttttccggt ttctacatgc agagatgaat ttatactgcc
2341 atcttacgac tatttcttct tttaatacac ttaactcagg ccattttttt aagttggtta
2401 cttcaaagta aataaacttt aaaattcaaa aaaaaaaaaa aaaaa
SEQ ID NO: 4: The protein sequence for Homo sapiens heat shock 70k Da protein 1B (HSPA1B_HUMAN) (NM_005346.4 / UniProtKB - P0DMV9): 
MAKAAAIGIDLGTTYSCVGVFQHGKVEIIANDQGNRTTPSYVAFTDTERLIGDAAKNQVALNPQNTVFDA

KRLIGRKFGDPVVQSDMKHWPFQVINDGDKPKVQVSYKGETKAFYPEEISSMVLTKMKEIAEAYLGYPVT

NAVITVPAYFNDSQRQATKDAGVIAGLNVLRIINEPTAAAIAYGLDRTGKGERNVLIFDLGGGTFDVSIL

TIDDGIFEVKATAGDTHLGGEDFDNRLVNHFVEEFKRKHKKDISQNKRAVRRLRTACERAKRTLSSSTQA
 
SLEIDSLFEGIDFYTSITRARFEELCSDLFRSTLEPVEKALRDAKLDKAQIHDLVLVGGSTRIPKVQKLL
QDFFNGRDLNKSINPDEAVAYGAAVQAAILMGDKSENVQDLLLLDVAPLSLGLETAGGVMTALIKRNSTI
PTKQTQIFTTYSDNQPGVLIQVYEGERAMTKDNNLLGRFELSGIPPAPRGVPQIEVTFDIDANGILNVTA
TDKSTGKANKITITNDKGRLSKEEIERMVQEAEKYKAEDEVQRERVSAKNALESYAFNMKSAVEDEGLKG
KISEADKKKVLDKCQEVISWLDANTLAEKDEFEHKRKELEQVCNPIISGLYQGAGGPGPGGFGAQGPKGG
SGSGPTIEEVD
SEQ ID NO: 5: The initiator methionine (M at position 1) of SEQ ID NO:4 is removed to yield a 640-amino acid long sequence (position 2-641): 
AKAAAIGIDLGTTYSCVGVFQHGKVEIIANDQGNRTTPSYVAFTDTERLIGDAAKNQVALNPQNTVFDAK
RLIGRKFGDPVVQSDMKHWPFQVINDGDKPKVQVSYKGETKAFYPEEISSMVLTKMKEIAEAYLGYPVTN
AVITVPAYFNDSQRQATKDAGVIAGLNVLRIINEPTAAAIAYGLDRTGKGERNVLIFDLGGGTFDVSILT
IDDGIFEVKATAGDTHLGGEDFDNRLVNHFVEEFKRKHKKDISQNKRAVRRLRTACERAKRTLSSSTQAS
LEIDSLFEGIDFYTSITRARFEELCSDLFRSTLEPVEKALRDAKLDKAQIHDLVLVGGSTRIPKVQKLLQ
DFFNGRDLNKSINPDEAVAYGAAVQAAILMGDKSENVQDLLLLDVAPLSLGLETAGGVMTALIKRNSTIP
TKQTQIFTTYSDNQPGVLIQVYEGERAMTKDNNLLGRFELSGIPPAPRGVPQIEVTFDIDANGILNVTAT
DKSTGKANKITITNDKGRLSKEEIERMVQEAEKYKAEDEVQRERVSAKNALESYAFNMKSAVEDEGLKGK
ISEADKKKVLDKCQEVISWLDANTLAEKDEFEHKRKELEQVCNPIISGLYQGAGGPGPGGFGAQGPKGGS
GSGPTIEEVD
SEQ ID NO: 6 The nucleic acid (DNA) sequence for Homo sapiens heat shock 70kDa protein 1B (HSPA1B) (NM_005346.4):

1 ggaaaacggc cagcctgagg agctgctgcg agggtccgct tcgtctttcg agagtgactc
61 ccgcggtccc aaggctttcc agagcgaacc tgtgcggctg caggcaccgg cgtgttgagt
121 ttccggcgtt ccgaaggact gagctcttgt cgcggatccc gtccgccgtt tccagccccc
181 agtctcagag cggagcccac agagcagggc accggcatgg ccaaagccgc ggcgatcggc
241 atcgacctgg gcaccaccta ctcctgcgtg ggggtgttcc aacacggcaa ggtggagatc
301 atcgccaacg accagggcaa ccgcaccacc cccagctacg tggccttcac ggacaccgag
361 cggctcatcg gggatgcggc caagaaccag gtggcgctga acccgcagaa caccgtgttt
421 gacgcgaagc ggctgatcgg ccgcaagttc ggcgacccgg tggtgcagtc ggacatgaag
481 cactggcctt tccaggtgat caacgacgga gacaagccca aggtgcaggt gagctacaag
541 ggggagacca aggcattcta ccccgaggag atctcgtcca tggtgctgac caagatgaag
601 gagatcgccg aggcgtacct gggctacccg gtgaccaacg cggtgatcac cgtgccggcc
661 tacttcaacg actcgcagcg ccaggccacc aaggatgcgg gtgtgatcgc ggggctcaac
721 gtgctgcgga tcatcaacga gcccacggcc gccgccatcg cctacggcct ggacagaacg
781 ggcaaggggg agcgcaacgt gctcatcttt gacctgggcg ggggcacctt cgacgtgtcc
841 atcctgacga tcgacgacgg catcttcgag gtgaaggcca cggccgggga cacccacctg
901 ggtggggagg actttgacaa caggctggtg aaccacttcg tggaggagtt caagagaaaa
961 cacaagaagg acatcagcca gaacaagcga gccgtgaggc ggctgcgcac cgcctgcgag

1021 agggccaaga ggaccctgtc gtccagcacc caggccagcc tggagatcga ctccctgttt
1081 gagggcatcg acttctacac gtccatcacc agggcgaggt tcgaggagct gtgctccgac
1141 ctgttccgaa gcaccctgga gcccgtggag aaggctctgc gcgacgccaa gctggacaag
1201 gcccagattc acgacctggt cctggtcggg ggctccaccc gcatccccaa ggtgcagaag
1261 ctgctgcagg acttcttcaa cgggcgcgac ctgaacaaga gcatcaaccc cgacgaggct
1321 gtggcctacg gggcggcggt gcaggcggcc atcctgatgg gggacaagtc cgagaacgtg
1381 caggacctgc tgctgctgga cgtggctccc ctgtcgctgg ggctggagac ggccggaggc
1441 gtgatgactg ccctgatcaa gcgcaactcc accatcccca ccaagcagac gcagatcttc
1501 accacctact ccgacaacca acccggggtg ctgatccagg tgtacgaggg cgagagggcc
1561 atgacgaaag acaacaatct gttggggcgc ttcgagctga gcggcatccc tccggccccc
1621 aggggcgtgc cccagatcga ggtgaccttc gacatcgatg ccaacggcat cctgaacgtc
1681 acggccacgg acaagagcac cggcaaggcc aacaagatca ccatcaccaa cgacaagggc
1741 cgcctgagca aggaggagat cgagcgcatg gtgcaggagg cggagaagta caaagcggag
1801 gacgaggtgc agcgcgagag ggtgtcagcc aagaacgccc tggagtccta cgccttcaac
1861 atgaagagcg ccgtggagga tgaggggctc aagggcaaga tcagcgaggc ggacaagaag
1921 aaggttctgg acaagtgtca agaggtcatc tcgtggctgg acgccaacac cttggccgag
1981 aaggacgagt ttgagcacaa gaggaaggag ctggagcagg tgtgtaaccc catcatcagc
2041 ggactgtacc agggtgccgg tggtcccggg cctggcggct tcggggctca gggtcccaag
2101 ggagggtctg ggtcaggccc taccattgag gaggtggatt aggggccttt gttctttagt
2161 atgtttgtct ttgaggtgga ctgttgggac tcaaggactt tgctgctgtt ttcctatgtc
2221 atttctgctt cagctctttg ctgcttcact tctttgtaaa gttgtaacct gatggtaatt
2281 agctggcttc attatttttg tagtacaacc gatatgttca ttagaattct ttgcatttaa
2341 tgttgatact gtaagggtgt ttcgttccct ttaaatgaat caacactgcc accttctgta
2401 cgagtttgtt tgtttttttt tttttttttt ttttttgctt ggcgaaaaca ctacaaaggc
2461 tgggaatgta tgtttttata atttgtttat ttaaatatga aaaataaaat gttaaacttt
2521 aaaaaaaaaa aaaaaaaaaa aaaaaaaaaa a

### Items

1. A method of detecting Hsp70 in a peripheral blood mononuclear cell (PBMC) sample, said method comprising the steps of
   a) providing a PBMC sample, and
   b) detecting Hsp70 in said PBMC sample, and
   c) optionally quantifying or determining the level of Hsp70 in said PBMC sample.
2. A method for diagnosing a disease presenting with a reduced level of Hsp70 in an individual, said method comprising the steps of:
   a) providing a PBMC sample from said individual,
   b) detecting Hsp70 in said PBMC sample, and
   c) quantifying or determining the level of Hsp70 in said PBMC sample.
3. The method according to item 2 further comprising the step of
   i. classifying or determining whether or not the individual has, or is likely to have, a disease presenting with a reduced level of Hsp70.
4. A method for selecting a patient having a disease presenting with a reduced level of Hsp70, said method comprising the steps of
   a) providing a PBMC sample from said patient,
   b) detecting Hsp70 in said PBMC sample,
   c) quantifying or determining the level of Hsp70 in said PBMC sample, and
   d) classifying or determining whether or not the individual has reduced levels of Hsp70.
5. The method according to any one of the preceding items which is an *in vitro* method.
6. The method according to any one of the preceding items comprising detecting and optionally quantifying or determining Hsp70 selected from
   i. HspA1A,
   ii. HspA1B, or
   iii. HspA1A and HspA1B.
7. The method according to any one of the preceding items, wherein said PBMC sample is obtained from or obtainable from an individual.
8. The method according to any one of the preceding items, wherein said individual has, is suspected of having, is at risk of having or is likely to have, a disease presenting with a reduced level of Hsp70.
9. The method according to any one of the preceding items, wherein said individual has one or more family members diagnosed with a disease presenting with a reduced level of Hsp70; such as one or more of a sibling, a parent, a cousin, an uncle and/or an aunt diagnosed with a disease presenting with a reduced level of Hsp70.
10. The method according to any one of the preceding items, wherein said individual has one or more family members with a genetic predisposition for a disease presenting with a reduced level of Hsp70*.*
11. The method according to any one of the preceding items, wherein said individual has one or more symptoms associated with or indicative of a disease presenting with a reduced level of Hsp70.
12. The method according to any one of the preceding items, wherein said step d) comprises determining the level of Hsp70 in the PBMC sample as compared to the levels in a PBMC sample from a healthy control.
13. The method according to any one of the preceding items, wherein
   i. a reduced or undetectable level of Hsp70 in the PBMC sample as compared to levels in a healthy control is indicative of the individual having, likely to have or at risk of having a disease presenting with a reduced level of Hsp70; and/or
   ii. a reduced or undetectable level of HspA1A in the PBMC sample as compared to levels in a healthy control is indicative of the individual having, likely to have or at risk of having a disease presenting with a reduced level of Hsp70; and/or
   iii. a reduced or undetectable level of HspA1B in the PBMC sample as compared to levels in a healthy control is indicative of the individual having, likely to have or at risk of having disease presenting with a reduced level of Hsp70, and/or
   iv. a level of Hsp70, HspA1A and/or HspA1B in the sample which is comparable to, equal to or higher than the level in a healthy control is indicative of the individual not having a disease presenting with a reduced level of Hsp70.
14. The method according to any one of the preceding items, wherein the individual is likely to have a disease presenting with a reduced level of Hsp70 if
   i. the level of Hsp70 in the PBMC sample is 1 to 1000 times lower than the level found in healthy controls, such as 1 to 2 times, 2 to 3 times, 3 to 4 times, 4 to 5 times, 5 to 6 times, 6 to 7 times, 7 to 8 times, 8 to 9 times, 9 to 10 times, 10 to 11 times, 11 to 12 times, 12 to 13 times, 13 to 14 times, 14 to 15 times, 15 to 16 times, 16 to 17 times, 17 to 18 times, 18 to 19 times, 19 to 20 times, 20 to 25 times, 25 to 30 times, 30 to 35 times, 35 to 40 times, 40 to 45 times, 45 to 50 times, 50 to 75 times, 75 to 100 times, 100 to 150 times, 150 to 200 times, 200 to 250 times, 250 to 300 times, 300 to 400 times, 400 to 500 times, 500 to 750 times, 750 to 1000 times lower than the level found in a healthy control, or undetectable, and/or
   ii. the level of HspA1A and/or HspA1B in the PBMC sample is 1 to 1000 times lower than the level found in healthy controls, such as 1 to 2 times, 2 to 3 times, 3 to 4 times, 4 to 5 times, 5 to 6 times, 6 to 7 times, 7 to 8 times, 8 to 9 times, 9 to 10 times, 10 to 11 times, 11 to 12 times, 12 to 13 times, 13 to 14 times, 14 to 15 times, 15 to 16 times, 16 to 17 times, 17 to 18 times, 18 to 19 times, 19 to 20 times, 20 to 25 times, 25 to 30 times, 30 to 35 times, 35 to 40 times, 40 to 45 times, 45 to 50 times, 50 to 75 times, 75 to 100 times, 100 to 150 times, 150 to 200 times, 200 to 250 times, 250 to 300 times, 300 to 400 times, 400 to 500 times, 500 to 750 times, 750 to 1000 times lower than the level found in a healthy control, or undetectable.
15. The method according to any one of the preceding items, wherein said step d) of classifying or determining an individual as having, or likely to have, a disease presenting with a reduced level of Hsp70 comprises
   i. determining if the amount of Hsp70 in said PBMC sample is below a predefined cut-off value, or undetectable; and/or
   ii. determining if the amount of HspA1A and/or HspA1B in said PBMC sample is below a predefined cut-off value, or undetectable.
16. The method according to any one of the preceding items, wherein the individual has or is likely to have a disease presenting with a reduced level of Hsp70 if the amount of Hsp70 in said PBMC sample is 7500 pg/mL or less, such as 7000 pg/mL or less, such as 6500 pg/mL or less, such as 6000 pg/mL or less, such as 5500 pg/mL or less, such as 5000 pg/mL or less, such as 4500 pg/mL or less, such as 4000 pg/mL or less, such as 3500 pg/mL or less, such as 3000 pg/mL or less, such as 2500 pg/mL or less, such as 2000 pg/mL or less, such as 1500 pg/mL or less, such as 1000 pg/mL PBMC or less.
17. The method according to any one of items 4-12, wherein the step of classifying or determining whether or not the patient has reduced levels of Hsp70 comprises a step of identifying a patient with reduced levels of Hsp70.
18. The method according to any one of items 4-12 and 17, wherein the step of classifying or determining whether or not the patient has reduced levels of Hsp70 comprises determining the level of Hsp70 in the PBMC sample as compared to the levels in a PBMC sample obtained or obtainable from a patient presenting with the same underlying disease but not having accompanying reduced levels of Hsp70.
19. The method according to any one of items 4-12 and 17-18, wherein said step d) of classifying or determining whether or not the patient has reduced levels of Hsp70, comprises
   i. determining if the amount of Hsp70 in said PBMC sample is below a predefined cut-off value, or undetectable; and/or
   ii. determining if the amount of HspA1A and/or HspA1B in said PBMC sample is below a predefined cut-off value, or undetectable.
20. The method according to any one of items 4-12 and 17-19, wherein the patient is likely, or more likely, to respond to Hsp70 therapies including bioactive agents that increase the intracellular concentration and/or activity of heat shock proteins, including Hsp70, if the amount of Hsp70 is below said cut-off value.
21. The method according to any one of items 4-12 and 17-20, wherein the patient presenting with a reduced level of Hsp70 is likely, or more likely, to respond to Hsp70 therapies if the amount of Hsp70 (such as HspA1A and/or HspA1B) in said PBMC sample is 7500 pg/mL or less, such as 7000 pg/mL or less, such as 6500 pg/mL or less, such as 6000 pg/mL or less, such as 5500 pg/mL or less, such as 5000 pg/mL or less, such as 4500 pg/mL or less, such as 4000 pg/mL or less, such as 3500 pg/mL or less, such as 3000 pg/mL or less, such as 2500 pg/mL or less, such as 2000 pg/mL or less, such as 1500 pg/mL or less, such as 1000 pg/mL PBMC or less.
22. The method according to any one of items 4-12 and 17-21 further comprising a step of determining eligibility of said patient for administering a therapy for treatment of said disease presenting with a reduced level of Hsp70 to the patient, such as Hsp70 therapies including bioactive agents that increase the intracellular concentration and/or activity of heat shock proteins, including Hsp70.
23. The method according to any one of the preceding items further comprising the step of
   i. administering a therapy for treatment of a disease presenting with a reduced level of Hsp70.
24. The method according to any one of the preceding items, wherein step a) providing a PBMC sample from an individual, comprise one or more steps of
   i. providing a whole blood sample from an individual, and
   ii. separating whole blood into its subcomponents to obtain a PBMC sample.
25. A method for monitoring disease progression in an individual having a disease presenting with a reduced level of Hsp70, said method comprising the steps of
   i. providing one or more PBMC samples from said individual at two or more subsequent points in time,
   ii. detecting Hsp70 in each of said PBMC samples,
   iii. quantifying or determining the level of Hsp70 in each of said PBMC samples.
26. The method according to item 25, wherein a first PBMC sample is taken at t=0 and one or more subsequent PBMC samples are taken at one or more later time points at t>0.
27. The method according to any one of items 25-26, wherein one or more subsequent samples are taken at an interval of 1 day, 2 days, 3 days, 4 days, 5 days, 6 days, 7 days, 14 days, 3 weeks, 4 weeks, 5 weeks, 6 weeks, 7 weeks, 8 weeks, 1 month, 2 months, 3 months, 4 months, 5 months, 6 months, 7 months, 8 months, 9 months, 10 months, 11 months and/or 12 months.
28. The method according to any one of items 25-27, said method further comprising the step of d) determining whether the disease presenting with a reduced level of Hsp70 is in progression or in remission.
29. The method according to any one of items 25-28, wherein
   a) a decrease in the level of Hsp70 over time is indicative of a progression of the disease; and/or
   b) an increase in the level of Hsp70 over time is indicative of a remission of the disease.
30. A method for monitoring efficacy of a therapy for treatment of a disease presenting with a reduced level of Hsp70 in an individual having a disease presenting with a reduced level of Hsp70, said method comprising the steps of
   a) providing one or more PBMC samples from said individual before, during and/or after a therapy has been applied, maintained, reduced or elevated,
   b) detecting Hsp70 in each of said one or more PBMC samples,
   c) quantifying or determining the level of Hsp70 in each of said one or more PBMC samples.
31. The method according to item 30, wherein
   i. one or more PBMC samples are obtained from an individual having a disease presenting with a reduced level of Hsp70 before a therapy has been applied, maintained, reduced or elevated;
   ii. one or more PBMC samples are obtained from an individual having a disease presenting with a reduced level of Hsp70 during a therapy; and/or
   iii. one or more PBMC samples are obtained from an individual having a disease presenting with a reduced level of Hsp70 after a therapy has been applied, maintained, reduced or elevated; and/or;
   iv. one or more PBMC samples are obtained from an individual having a disease presenting with a reduced level of Hsp70 before, during and/or after a therapy has been applied, maintained, reduced or elevated.
32. The method according to any one of items 30-31, said method further comprising the step of d) monitoring efficacy of a therapy for a disease presenting with a reduced level of Hsp70.
33. The method according to any one of items 30-32, wherein
   a) an increase in the level of Hsp70, after a therapy has been applied, maintained, reduced or elevated, is indicative of the therapy being efficacious; and/or
   b) a decrease in the level of Hsp70, after a therapy has been applied, maintained, reduced or elevated, is indicative of the therapy being inefficacious.
34. The method according to any one of the preceding items, wherein said detecting Hsp70 comprises detecting Hsp70 protein or RNA, such as Hsp70 protein.
35. The method according to any one of the preceding items, wherein said Hsp70 is detected and quantified by means of enzyme-linked immunosorbent assay (ELISA).
36. The method according to any one of the preceding items, wherein step c) quantifying or determining the level Hsp70 in a sample comprises one or more steps of
   i. normalizing to a calibrated standard, and
   ii. quantification of Hsp70 in said PBMC sample.
37. The method according to any one of the preceding items, wherein a therapy for treatment of a disease presenting with a reduced level of Hsp70 is a bioactive agent that increase the intracellular concentration and/or activity of heat shock proteins, including Hsp70.
38. The method according to any one of the preceding items, wherein said bioactive agent that increase the intracellular concentration and/or activity of heat shock proteins, including Hsp70, is selected from Hsp70 protein, or a functional fragment or variant thereof, a heat shock protein inducer and a Hsp70 inducer or co-inducer.
39. The method according to any one of the preceding items, wherein said bioactive agent that increase the intracellular concentration and/or activity of heat shock proteins, including Hsp70, is a small molecule hydroxylamine derivative capable of increasing the intracellular concentration (or levels) of Hsp70 by amplifying Hsp70 gene expression.
40. The method according to any one of the preceding items, wherein said bioactive agent that increases the intracellular concentration and/or activity of heat shock proteins, including Hsp70, is selected from the group consisting of arimoclomol, iroxanadine, bimoclomol, BGP-15, their stereoisomers and the acid addition salts thereof
41. The method according to any one of the preceding items, wherein said disease presenting with a reduced level of Hsp70 is a lysosomal storage disease, a neurodegenerative disease, a neuromuscular disorder, muscular dystrophy and an inflammatory muscle disorder.
42. The method according to any one of the preceding items, wherein said disease presenting with a reduced level of Hsp70 is a lysosomal storage disease.
43. The method according to item 42, wherein said lysosomal storage disease is selected from the group consisting of a lipid storage disorder including the sphingolipidoses; mucopolysaccharidoses; glycogen storage disorders; disorders of glycoprotein metabolism (glycoproteinosis); and mucolipidoses.
44. The method according to item 43, wherein said lysosomal storage disease is a sphingolipidosis.
45. The method according to item 42, wherein said lysosomal storage disease is selected from the group consisting of Niemann Pick disease (including types A, B and C), Farber disease, Krabbe disease, Fabry disease, Gaucher disease, Sialidosis (Mucolipidosis type I), sulfatidosis including Metachromatic leukodystrophy (late infantile, juvenile, and adult forms), saposin-deficiency, Multiple sulfatase deficiency (Austin disease), Gaucher disease (including type I, type II and type III), cerebrotendinous cholesterosis, Wolman's disease (Lysosomal acid lipase deficiency), cholesteryl ester storage disease, neuronal ceroid lipofuscinosis (NCL, including Batten disease (Spielmeyer-Vogt disease), Bielschowsky-Jansky disease, Kufs disease, Santavuori-Haltia disease), mucopolysaccharidosis (type I, type II, type III, type IV, type VI, type VII, type VIII and type IX), mucolipidosis (type II, type III and type IV),cardiac glycogenosis, Andersen disease, Cori disease (Forbes disease), Hers disease, McArdle disease, Pompe disease, Tauri disease (Tarui disease), von Gierke disease, type II Pompe disease, type IIb Danon disease,aspartylglucosaminuria, fucosidosis, annosidosis, alpha-mannosidosis, alpha-mannosidosis type I, alpha-mannosidosis type II, beta-mannosidosis, sialidosis type II (mucolipidosis I) and galactosialidosis.
46. The method according to item 42, wherein said lysosomal storage disease is Niemann Pick disease, such as Niemann Pick disease Type C.
47. The method according to any one of the preceding items, wherein said disease presenting with a reduced level of Hsp70 is a neurodegenerative disease.
48. The method according to item 47, wherein said neurodegenerative disease is selected from the group consisting of Parkinson's disease, Alzheimer's disease, Amyotrophic lateral sclerosis (ALS), Multiple Sclerosis, Huntington's disease, polyglutamine diseases and the spinocerebellar ataxias including Spinocerebellar ataxia type 1, Spinocerebellar ataxia type 2, Spinocerebellar ataxia type 3 (aka Machado-Joseph's disease), Spinocerebellar ataxia type 6, Spinocerebellar ataxia type 7 and Spinocerebellar ataxia type 17), DRPLA (Dentatorubropallidoluysian atrophy) and SBMA (Spinobulbar muscular atrophy or Kennedy disease).
49. The method according to any one of the preceding items, wherein said disease presenting with a reduced level of Hsp70 is selected from the group consisting of a neuromuscular disorder, muscular dystrophy and an inflammatory muscle disorder.
50. The method according to item 49, wherein said neuromuscular disorder is selected from the group consisting of Amyotrophic lateral sclerosis (ALS), Multiple Sclerosis, Parkinson's disease, Huntington's disease, Creutzfeldt-Jakob disease, Myasthenia gravis, Spinal Muscular Atrophy (SMA), Spinal muscular atrophy with respiratory distress type 1 (SMARD1; aka. Distal spinal muscular atrophy type 1 (DSMA1)), Congenital myasthenic syndrome (CMS), Congenital myopathy, Cramp fasciculation syndrome, Muscular dystrophies, Hereditary spastic paraplegia, Inclusion body myositis, Neuromyotonia (NMT, aka Isaacs syndrome, Isaacs-Merton syndrome), Mitochondrial myopathy, Lambert-Eaton myasthenic syndrome (LEMS), Myotonic dystrophy, Peripheral neuropathy, Spinal and bulbar muscular atrophy (SBMA, or Kennedy's disease), Stiff person syndrome and Guillain-Barré syndrome.
51. The method according to item 49, wherein said Muscular dystrophy is selected from the group consisting of Duchenne muscular dystrophy (DMD), Becker muscular dystrophy, Congenital muscular dystrophy, Distal muscular dystrophy, Emery-Dreifuss muscular dystrophy, Facioscapulohumeral muscular dystrophy, Limb-girdle muscular dystrophy, Myotonic muscular dystrophy and Oculopharyngeal muscular dystrophy.
52. The method according to item 49, wherein said inflammatory muscle disorder is selected from the group consisting of Inflammatory myopathy (inflammatory muscle disease or myositis), idiopathic Inflammatory myopathy, Polymyositis (PM) , dermatomyositis (DM), Inclusion-body myositis (sIBM and hIBM), Polymyalgia rheumatica (or "muscle rheumatism") and Rhabdomyolysis.
53. A method for adjusting dosage of a small molecule hydroxylamine derivative capable of increasing the intracellular concentration (or levels) of Hsp70 in an individual by amplifying Hsp70 gene expression, the method comprising the steps of:
   a) providing a PBMC sample from said individual,
   b) detecting Hsp70 in said PBMC sample,
   c) quantifying or determining a first level of Hsp70 in said PBMC sample,
   d) administering said small molecule hydroxylamine derivative at a first dose,
   e) repeating steps a) - c) to identify a second level of Hsp70 in said PBMC sample,
   f) comparing said first level to the second level of Hsp70, and
   g) optionally adjusting the dosage of the small molecule hydroxylamine derivative based on said comparison.

### Examples

### Example 1: Determining Hsp70 level in PBMC samples

The level of Hsp70 protein in PBMC samples isolated from healthy individuals was compared to Hsp70 levels in PBMC samples from individuals with Niemann Pick disease Type C.

### Introduction

Niemann Pick disease Type C (NPC) is a rare devastating neurodegenerative disease, caused by mutations in either the NPC1 (95% of cases) or NPC2 genes. While NPC1 is a lysosomal/endosomal membrane protein, NPC2 is a soluble cholesterol binding lysosomal protein, and together they play essential roles in lysosomal biogenesis. NPC disease is characterized by an enlarged, dysfunctional lysosomal compartment and aberrant accumulation of cholesterol and glycosphingolipids (GSL) inside the cells of multiple tissues causing the pathology of the disease (Platt, 2014).

Hsp70, an evolutionary conserved protein, stabilizes the lysosome by directed interaction with lysosomal proteins (T Kirkegaard et al., 2010). Therefore, Hsp70 therapies were considered as a potential treatment for NPC. Correspondingly, administration of Hsp70 or arimoclomol, a well-known inducer of the heat shock response, reduced the size of the lysosomal compartment and reversed the neuronal as well as visceral pathology of mutant NPC1 mice (Thomas Kirkegaard et al., 2016). Furthermore, the endogenous level of Hsp70 was found to be reduced in the brain and liver of the NPC1 mice.

A prospective non-interventional clinical study in NPC patients was initiated prior to initiation of a placebo controlled study with arimoclomol. Patients, 2 to 18 years of age, diagnosed with NPC, who had at least one neurological symptom and who had preserved ability to walk with assistance were eligible. Patients were maintained on standard therapy. All data were evaluated at inclusion (visit 1) and after 6 to 14 months (visit 2) of prospective observation. Disease severity was determined using the NPC-severity scale score (Yanjanin et al., 2010), where a higher score is associated with a more severe disease.

To gain a better understanding the disease pathology, we set out to determine Hsp70 in untreated patients. However, neither brain nor liver is an appropriate matrix for NPC patients. At the time of the planning of the clinical study a paper describing the classical NPC pathology in PBMC had just been published (Te Vruchte et al., 2014). We therefore found PBMC as a relevant matrix for determination of Hsp70 in NPC patients.

### Methods

### Sample collection:

Whole blood samples were collected in 6 mL EDTA tubes at the clinical study sites and shipped ambient to a central laboratory for sample preparation.

Clinical study samples (n=26) were obtained from individuals with Niemann Pick disease Type C (NPC patients), who were 2 to 18 years of age, diagnosed with NPC, had at least one neurological symptom and had preserved ability to walk with assistance. Patients were maintained on standard therapy during the study. Samples were collected at a first clinical study visit (inclusion) and at a second clinical study visit 6 to 14 months following the first visit.

Control samples (n=19) were obtained from healthy subjects at age 20 to 30. These samples were handled using the same collection and separation methods as the clinical study samples.

### NPC-severity scale score:

At the first and second clinical study visit, disease severity of NPC patients was determined using the NPC-severity scale score (NPCCSS) (Yanjanin et al., 2010), where a higher score is associated with a more severe disease.

### Separation of PBMCs:

Plasma was separated from whole blood by centrifugation at room temperature. Blood volume was restored in PBS and PBMCs were isolated by density gradient centrifugation using Histopaque^{™}. Viable PBMC cell count was determined by flow cytometry. Aliquots of 1 mio. PBMC were generated and stored frozen at -70 °C.

### Determination of Hsp70 in human PBMC:

Hsp70 was quantified using the Human/Mouse/Rat total HSP70 DuoSet^{®} IC ELISA kit (R&D Systems). Prior to analysis of the clinical study samples, the analytical method had been qualified for determination of Hsp70 in human PBMC samples. The Human/Mouse/Rat total HSP70 DuoSet^{®} IC ELISA kit detects the levels of HspA1A and HspA1B with no cross-reactivity of HspA5 and HspA8.

### Results

Quantification of Hsp70 in the PBMC samples showed a markedly reduced expression of Hsp70 in the PBMC samples derived from NPC patients compared to the PBMC samples from healthy controls. The healthy control samples showed an average of 12000 pg/mL of Hsp70, whereas the average concentration of Hsp70 in the PBMC samples isolated from NPC patients was 1800 pg/mL (Figure 1). The expression of Hsp70 in PBMC samples isolated from NPC patients showed no correlation with the NPC-severity scale score (NPCCSS) (figure 2). Furthermore, no change in Hsp70 level over the time from clinical study visit 1 to visit 2 was observed (Figure 3).

### Conclusion

The example demonstrates that Hsp70 protein expression is markedly reduced in Peripheral Blood mononuclear cells (PBMC) in patients suffering from Niemann Pick Type C as compared to healthy controls. Furthermore, the low level of Hsp70 is independent of the disease severity of the NPC patients.

### Example 2: Arimoclomol increases Hsp70 in NPC patients

### Materials & Methods

The materials and methods were performed as described in Example 1.

### Results

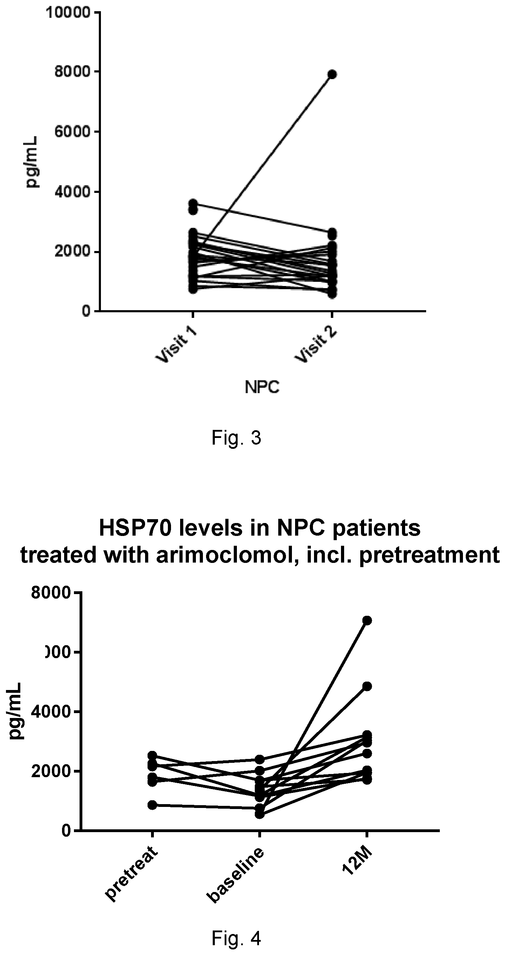

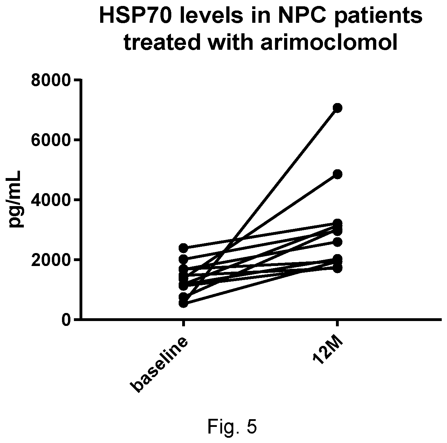

The Hsp70 concentration was measured in homogenates of Peripheral Blood Mononuclear Cells (PBMC). Hsp70 was determined in an observational study (Figures 4 and 5), at screening (pretreatment) and then again after 6-14 months (baseline). Patients were treated for 12 months with arimoclomol (Clinicaltrials.gov identifier NCT02612129). Hsp70 levels were analyzed by the Wilcoxon signed rank test for the 12 patients on arimoclomol with baseline and 12 months data point comparing the Hsp70 levels before and after 12 months of treatment.

**Table 2: Statistical analysis of retrieved data.**

| Change from baseline (pg/mL) arimoclomol | |
|---|---|
| n | 12 |
| mean (SD) | 1815.0 (1754.6) |
| Median | 1175.5 |
| Min - max | 234.7-6509.0 |
| Paired t-test p-value | 0.0043 |
| Wilcoxon signed rank test p-value | 0.0005 |

### Conclusion

Hsp70 blood levels are reduced in patients with NPC, compared to healthy controls, and remains stable with disease progression (from pretreat to baseline). This example demonstrates that treatment with arimoclomol for 12 months significantly increased the PBMC-level of Hsp70 compared to baseline. This demonstrates that the PBMC-level of Hsp70 is a pharmacodynamic marker for arimoclomol therapy.

### References

Kirkegaard, T., Gray, J., Priestman, D. A., Wallom, K., Atkins, J., Olsen, O. D., ... Platt, F. M. (2016). Heat shock protein-based therapy as a potential candidate for treating the sphingolipidoses. Science Translational Medicine, 8(355), 355ra118. https://doi.org/10.1126/scitranslmed.aad9823
Kirkegaard, T., Roth, A. G., Petersen, N. H. T., Mahalka, A. K., Olsen, O. D., Moilanen, I., ... Jäättelä, M. (2010). Hsp70 stabilizes lysosomes and reverts Niemann-Pick disease-associated lysosomal pathology. Nature, 463(7280), 549-53. https://doi.org/10.1038/nature08710
Platt, F. M. (2014). Sphingolipid lysosomal storage disorders. Nature, 510(7503), 68-75. https://doi.org/10.1038/nature13476
Te Vruchte, D., Speak, A. O., Wallom, K. L., Al Eisa, N., Smith, D. A., Hendriksz, C. J., ... Platt, F. M. (2014). Relative acidic compartment volume as a lysosomal storage disorder-associated biomarker. The Journal of Clinical Investigation, 1-9. https://doi.org/10.1172/JCI72835
Yanjanin, N. M., Vélez, J. I., Sc, M., Gropman, A., King, K., Au, D., ... Porter, F. D. (2010). Linear Clinical Progression, Independent of Age of Onset, in Niemann-Pick Disease, type C. American Journal Of Medical Genetics Part B Neuropsychiatric Genetics, (1), 132-140. https://doi.org/10.1002/ajmg.b.30969.Linear

## Claims

1. A kit for detecting and quantifying Hsp70 in a peripheral blood mononuclear cell (PBMC) sample, wherein said Hsp70 is HspA1A and/or HspA1B,
the kit comprising a means for detecting and quantifying the level of said Hsp70 in the PBMC sample,
such as wherein the kit comprises a PBMC sample comprising Hsp70, and a means for detecting and quantifying the level of the Hsp70 in the sample.

2. The kit according to claim 1, wherein the means for detecting and quantifying the level of said Hsp70 comprises:
a. a primary antibody capable of binding to HspA1A and/or HspA1B, and
b. a secondary antibody capable of binding to the primary antibody.

3. The kit according to claim 2, wherein the primary antibody binds to HspA1A and/or HspA1B with no or little cross reactivity with a Heat-shock protein selected from HspA5 and HspA8.

4. The kit according to claim 1, wherein the means for detecting and quantifying the level of said Hsp70 comprises one or more substrates for visualizing the level of the Hsp70 in the sample, such as colorimetric or chemiluminescent detection.

5. The kit according to claim 1, wherein the means for detecting and quantifying the level of said Hsp70 in the sample is selected from High-performance liquid chromatography (HPLC) and Liquid chromatography-mass spectrometry (LC/MS).

6. The kit according to claim 1, wherein the means for detecting and quantifying the level of said Hsp70 is performed on a method selected from enzyme-linked immunosorbent assay (ELISA), western blotting, Protein immunoprecipitation, Immunoelectrophoresis, Protein immunostaining, High-performance liquid chromatography (HPLC) and Liquid chromatography-mass spectrometry (LC/MS).

7. The kit of claim 1, wherein the means for detecting and quantifying the level of said Hsp70 detects a nucleotide coding for HspA1A and/or HspA1B, such as wherein the nucleotide coding for HspA1A and/or HspA1B is RNA.

8. A method of detecting Hsp70 in a peripheral blood mononuclear cell (PBMC) sample, said method comprising the steps of
a. providing a PBMC sample, and
b. detecting Hsp70 in said PBMC sample, and
c. optionally quantifying or determining the level of Hsp70 in said PBMC sample.

9. The kit or the method according to any one of the preceding claims, wherein the PBMC sample is obtained from a patient that has or is likely to have a disease presenting with a reduced level of Hsp70,
such as a lysosomal storage disorder,
such as a lysosomal storage disorder selected from Niemann Pick disease (including types A, B and C), Farber disease, Krabbe disease, Fabry disease, Gaucher disease, Sialidosis (Mucolipidosis type I), sulfatidosis including Metachromatic leukodystrophy (late infantile, juvenile, and adult forms), saposin-deficiency, Multiple sulfatase deficiency (Austin disease), Gaucher disease (including type I, type II and type III), cerebrotendinous cholesterosis, Wolman's disease (Lysosomal acid lipase deficiency), cholesteryl ester storage disease, neuronal ceroid lipofuscinosis (NCL, including Batten disease (Spielmeyer-Vogt disease), Bielschowsky-Jansky disease, Kufs disease, Santavuori-Haltia disease), mucopolysaccharidosis (type I, type II, type III, type IV, type VI, type VII, type VIII and type IX), mucolipidosis (type II, type III and type IV), cardiac glycogenosis, Andersen disease, Cori disease (Forbes disease), Hers disease, McArdle disease, Pompe disease, Tauri disease (Tarui disease), von Gierke disease, type II Pompe disease, type lib Danon disease, aspartylglucosaminuria, fucosidosis, annosidosis, alpha-mannosidosis, alpha-mannosidosis type I, alpha-mannosidosis type II, beta-mannosidosis, sialidosis type II (mucolipidosis I) and galactosialidosis.

10. The kit or the method according to claim any one of the preceding claims, wherein the PBMC sample is obtained from a patient that has or is likely to have a neurodegenerative disease or neuromuscular disorder, such as amyotrophic lateral sclerosis (ALS).

11. The kit or the method according to any one of the preceding claims, wherein the kit or the method indicates a patient has or is likely to have a disease presenting with a reduced level of Hsp70, such as a lysosomal storage disorder or ALS, if
a. the detected level of Hsp70 in the PBMC sample is 1 to 1000 times lower than the level found in healthy controls, such as 1 to 2 times, 2 to 3 times, 3 to 4 times, 4 to 5 times, 5 to 6 times, 6 to 7 times, 7 to 8 times, 8 to 9 times, 9 to 10 times, 10 to 1 1 times, 1 1 to 12 times, 12 to 13 times, 13 to 14 times, 14 to 15 times, 15 to 16 times, 16 to 17 times, 17 to 18 times, 18 to 19 times, 19 to 20 times, 20 to 25 times, 25 to 30 times, 30 to 35 times, 35 to 40 times, 40 to 45 times, 45 to 50 times, 50 to 75 times, 75 to 100 times, 100 to 150 times, 150 to 200 times, 200 to 250 times, 250 to 300 times, 300 to 400 times, 400 to 500 times, 500 to 750 times, 750 to 1000 times lower than the level found in a healthy control, or undetectable, and/or
b. the detected level of HspA1 A and/or HspA1 B in the PBMC sample is 1 to 1000 times lower than the level found in healthy controls, such as 1 to 2 times, 2 to 3 times, 3 to 4 times, 4 to 5 times, 5 to 6 times, 6 to 7 times, 7 to 8 times, 8 to 9 times, 9 to 10 times, 10 to 1 1 times, 1 1 to 12 times, 12 to 13 times, 13 to 14 times, 14 to 15 times, 15 to 16 times, 16 to 17 times, 17 to 18 times, 18 to 19 times, 19 to 20 times, 20 to 25 times, 25 to 30 times, 30 to 35 times, 35 to 40 times, 40 to 45 times, 45 to 50 times, 50 to 75 times, 75 to 100 times, 100 to 150 times, 150 to 200 times, 200 to 250 times, 250 to 300 times, 300 to 400 times, 400 to 500 times, 500 to 750 times, 750 to 1000 times lower than the level found in a healthy control, or undetectable.

12. The kit or the method of claim 11, wherein the patient has or is likely to have a disease presenting with a reduced level of Hsp70, such as a lysosomal storage disorder or ALS, if the amount of said Hsp70 in said PBMC sample is 7500 pg/mL or less, such as 7000 pg/mL or less, such as 6500 pg/mL or less, such as 6000 pg/mL or less, such as 5500 pg/mL or less, such as 5000 pg/mL or less, such as 4500 pg/mL or less, such as 4000 pg/mL or less, such as 3500 pg/mL or less, such as 3000 pg/mL or less, such as 2500 pg/mL or less, such as 2000 pg/mL or less, such as 1500 pg/mL or less, such as 1000 pg/mL or less.

13. A method of diagnosing a disease presenting with a reduced level of Hsp70 in an patient, said method comprising the steps of
a. providing a PBMC sample, and
b. detecting Hsp70 in said PBMC sample, and
c. optionally quantifying or determining the level of Hsp70 in said PBMC sample,
d. optionally classifying or determining whether or not the patient has, or is likely to have, a disease presenting with a reduced level of Hsp70.

14. A method for selecting a patient having a disease presenting with a reduced level of Hsp70 for treatment with a bioactive agent that increases the intracellular concentration and/or activity of heat shock proteins, including Hsp70, said method comprising the steps of
a. providing a PBMC sample, and
b. detecting Hsp70 in said PBMC sample, and
c. optionally quantifying or determining the level of Hsp70 in said PBMC sample,
d. optionally classifying or determining whether or not the patient has, or is likely to have, a disease presenting with a reduced level of Hsp70.

15. The method according to any one of claims 13 or 14, wherein the disease presenting with a reduced level of Hsp70 is a lysosomal storage disorder, such as NPC, or amyotrophic lateral sclerosis (ALS).
